# EUROPÄISCHE PATENTSCHRIFT

(11) **EP 2 628 455 B1**
(45) Veröffentlichungstag und Bekanntmachung des Hinweises auf die Patenterteilung: **27.07.2016**
(21) Anmeldenummer: 13155332.3
(22) Anmeldetag: 15.02.2013
(51) Int. Cl.: A61B 17/221, A61F 2/915

(54) **Medizinische Vorrichtung mit einer Gitterstruktur und Behandlungssystem mit einer derartigen medizinischen Vorrichtung**
Medical device having a mesh structure and treatment system with such a medical device
Dispositif médical doté d'une structure de grille et système de traitement équipé d'un tel dispositif médical

(30) Priorität: 17.02.2012 DE 102012101284
(43) Veröffentlichungstag der Anmeldung: 21.08.2013
(73) Patentinhaber: Acandis GmbH & Co. KG, 76327 Pfinztal (DE)
(72) Erfinder: Cattaneo, Giorgio, 76199 Karlsruhe (DE)
(74) Vertreter: Kilchert, Jochen

(56) Entgegenhaltungen:
- DE-A1-102009 041 025
- DE-A1-102010 045 367
- US-A1- 2006 058 837
- US-A1- 2012 215 250

## Beschreibung

Die Erfindung betrifft eine medizinische Vorrichtung mit einer komprimierbaren und expandierbaren, kreiszylinderförmigen Gitterstruktur, die Umfangssegmente aus geschlossenen Zellen umfasst, gemäß des Gegenstands von Anspruch 1.

Das technische Gebiet der Erfindung betrifft insbesondere stentähnliche Systeme bzw. Vorrichtungen zur Behandlung von Erkrankungen des Herz-Kreislaufsystems. Ein besonders bevorzugtes Gebiet sind Vorrichtungen zum Entfernen von Konkrementen aus Körperhohlorganen, insbesondere Thrombektomie-Devices.

Thrombektomie-Devices sind aus der Praxis allgemein bekannt und umfassen eine korbartige, insbesondere zumindest abschnittsweise kreiszylinderförmige, Gitterstruktur, die durch einen Zuführkatheter an den Behandlungsort geführt werden kann. Die Gitterstruktur ist expandierbar und komprimierbar, so dass die Gitterstruktur beim Vorschieben durch einen Zuführkatheter einen relativ kleineren Querschnittsdurchmesser als im expandierten Zustand aufweist, den die Gitterstruktur innerhalb eines Körperhohlorgans einnimmt. Die Gitterstruktur bekannter Systeme umfasst Stege, die Zellen der Gitterstruktur begrenzen. Beim Übergang vom komprimierten in den expandierten Zustand bewegen sich die Stege ausgehend von der Längsachse der Gitterstruktur radial geradlinig nach außen. Dabei können sich die Stege der bekannten Gitterstruktur in radialer Richtung bezogen auf die Längsachse der Gitterstruktur in ein Konkrement, insbesondere ein Blutgerinnsel, das in dem Körperhohlorgan angeordnet ist und beispielsweise eine Minderdurchblutung nachgeordneter Gewebeteile bewirkt, hineinschneiden. Auf diese Weise wird die bekannte Gitterstruktur mit dem Blutgerinnsel verbunden, so dass das Blutgerinnsel aus dem Körperhohlorgan extrahiert werden kann.

Es hat sich gezeigt, dass sich der Thrombus beim Entfernen des Konkrements aus dem Körperhohlorgan, insbesondere beim Ziehen der bekannten Vorrichtungen bzw. Thrombektomie-Devices durch ein Blutgefäß, von der Gitterstruktur lösen kann. Dieses Risiko besteht insbesondere beim Zurückziehen der Gitterstruktur in größere Blutgefäße, so dass sich der Thrombus in radialer Richtung nach außen von der Gitterstruktur entfernen kann. Bei den bekannten Thrombektomie-Devices besteht also die Gefahr, dass das Blutgerinnsel bzw. der Thrombus nicht ausreichend fest an der Gitterstruktur anhaftet.

DE102009041025 beschreibt eine medizinische Vorrichtung gemäß des Oberbegriffs von Anspruch 1, welche über, aus jeweils 4 Stegen bestehenden, rautenförmige Zellen verfügt, die ein radial nach außen auslenkbares Haltelement aufweisen, welches derart orientiert ist, dass seine Spitze in Längsrichtung der medizinischen Vorrichtung weist.

Die Aufgabe der Erfindung besteht darin, eine medizinische Vorrichtung mit einer komprimierbaren und expandierbaren, kreiszylinderförmigen Gitterstruktur anzugeben, die eine verbesserte Verankerung in einem Blutgerinnsel ermöglicht und eine gute Handhabbarkeit aufweist. Ferner besteht die Aufgabe der Erfindung darin, ein Behandlungssystem mit einer derartigen Vorrichtung anzugeben.

Erfindungsgemäß wird diese Aufgabe im Hinblick auf die medizinische Vorrichtung durch den Gegenstand des Patentanspruchs 1 und im Hinblick auf das Behandlungssystem durch den Gegenstand des Patentanspruchs 13 gelöst.

Die Erfindung beruht auf dem Gedanken, eine medizinische Vorrichtung mit einer komprimierbaren und expandierbaren, kreiszylinderförmigen Gitterstruktur anzugeben, die geschlossene Zellen umfasst. Die Zellen sind durch jeweils wenigstens vier Stege begrenzt, die an Verbindungsstellen miteinander gekoppelt sind. Die Verbindungsstellen bilden im expandierten Zustand der Gitterstruktur eine rautenförmige Grundform der Zelle. Weinigstens eine Zelle weist einen Zwischensteg auf, der sich zwischen zwei in Längsrichtung der Gitterstruktur direkt benachbarten und miteinander verbundenen Stege der Zelle durch die Zelle erstreckt und eine Spitze bildet, die im Wesentlichen in Umfangsrichtung der Gitterstruktur zeigt.

Bei der erfindungsgemäßen medizinischen Vorrichtung ist also vorgesehen, dass der Zwischensteg im Wesentlichen in Längsrichtung der medizinischen Vorrich-tung bzw. der Gitterstruktur ausgerichtet ist. Die durch den Zwischensteg gebildete Spitze weist dabei in Umfangsrichtung der Gitterstruktur. Mit anderen Worten kann der Zwischensteg zwei Abschnitte, insbesondere Verbindungselemente, aufweisen, die einerseits mit jeweils einem Steg der Zelle verbunden sind, wobei die Verbindung mit Stegen erfolgt, die in Längsrichtung der Gitterstruktur unmittelbar benachbart zueinander angeordnet und miteinander verbunden sind. Andererseits können die beiden Abschnitte bzw. Verbindungselemente zur Bildung des Zwischenstegs miteinander verbunden sein, wobei die beiden Abschnitte bzw. Verbindungselemente in Richtung der Kopplungsstelle sich zumindest teilweise in Umfangsrichtung erstrecken. Dies gilt für den expandierten Zustand.

Es wird darauf hingewiesen, dass im Rahmen der Anmeldung die Spitze nicht zwingend spitz zulaufend ausgebildet sein muss, sondern auch durch eine Krümmung des Zwischenstegs gebildet sein kann, die sich im Wesentlichen in-Umfangsrichtung der Gitterstruktur wölbt.

Der Vorteil der Zwischenstege besteht darin, dass sich die Zwischenstege beim Expandieren der Gitterstruktur radial nach außen aufstellen. Die Spitze des Zwischenstegs ragt also im expandierten Zustand über die Umfangsebene der Gitterstruktur vor. Somit kann sich die Gitterstruktur gut mit einem Blutgerinnsel verbinden. Konkret wird durch die sich nach außen aufstellende Spitze des Zwischenstegs erreicht, dass im expandierten Zustand der Gitterstruktur die Spitze des Zwischenstegs einen größeren Abstand zur Längsachse der Gitterstruktur aufweist als die Stege der zugehörigen Zelle. Die Stege der Zelle sind also in einer anderen Umfangsebene angeordnet als die Spitze des Zwischenstegs. Durch eine Rotation der Gitterstruktur kann nun in die zwischen der Spitze des Zwischenstegs und den Stegen der Zelle gebildete Lücke ein Konkrement bzw. ein Teil eines Konkrements eindringen. Somit können Blutgerinnsel oder dergleichen zwischen der Spitze des Zwischenstegs und den Stegen der Zelle fixiert werden. Die Verankerung der medizinischen Vorrichtung mit einem Blutgerinnsel oder dergleichen wird somit verbessert.

Bei einer bevorzugten Ausführungsform der erfindungsgemäßen medizinischen Vorrichtung ist vorgesehen, dass der Zwischensteg mit den beiden direkt benachbarten und miteinander verbundenen Stegen eine Unterzelle bildet, die im Wesentlichen dieselbe Form wie die übergeordnete Zelle aufweist. Mit anderen Worten folgt der Zwischensteg der Form zweier parallel angeordneter Stege der Zelle, mit denen der Zwischensteg nicht verbunden ist. Der Zwischensteg ist mit zwei Stegen der Zelle gekoppelt. Zwei weitere Stege der Zelle sind frei.

Der Zwischensteg weist vorzugsweise die Form der freien Stege auf, wobei der Zwischensteg kürzer als die beiden freien Stege ist. Durch die Geometrieanalogie zwischen dem Zwischensteg und den freien Stegen bzw. der durch den Zwischensteg gebildeten Unterzelle und der übergeordneten Zelle wird eine gleichmäßige Aufstellung der Spitze des Zwischenstegs im expandierten Zustand ermöglicht. Ferner werden durch diese, im Folgenden als symmetrisch bezeichnete, Form der Unterzelle die auf dem Zwischensteg beim Einschneiden in ein Blutgerinnsel oder dergleichen wirkenden Kräfte gleichmäßig auf die Stege der Zelle übertragen. Eine Verdrehung bzw. ein Ausweichen der Gitterstruktur wird dadurch vermieden.

Der Zwischensteg kann durch zwei Verbindungselemente gebildet sein, die im Bereich der Spitze miteinander verbunden sind. Die Verbindung zwischen den Verbindungselementen kann stoffschlüssig erfolgen. Vorzugsweise sind die Verbindungselemente unterschiedlich flexibel, so dass der Steg, der mit dem Verbindungselement mit niedrigerer Flexibilität verbunden ist, beim Übergang der Gitterstruktur vom expandierten Zustand in den komprimierten Zustand stärker verformbar ist als der Steg, der mit dem Verbindungselement mit höherer Flexibilität verbunden ist. Dabei können die Zellen eines Umfangssegments der Gitterstruktur gleichartig ausgebildet sein, so dass die gesamte Gitterstruktur beim Übergang vom expandierten Zustand in den komprimierten Zustand zumindest abschnittsweise tordiert.

Durch die unterschiedlichen Flexibilitäten der Verbindungselemente wird erreicht, dass, zusätzlich zur verbesserten Verankerung der Gitterstruktur mit einem Blutgerinnsel oder dergleichen, durch das Aufstellen der Spitze eine weitere Funktion bereitgestellt wird, nämlich die selbsttätige Torsion der Gitterstruktur während der Expansion. Die Gitterstruktur verdreht sich also in sich beim Expandieren, wodurch im Wesentlichen automatisch ein Eingriff der Gitterstruktur, insbesondere den Zwischenstegen, und einem Blutgerinnsel ermöglicht wird.

Gemäß einem nebengeordneten Aspekt beruht die Erfindung auf dem Gedanken, eine medizinische Vorrichtung mit einer komprimierbaren und expandierbaren, kreiszylinderförmigen Gitterstruktur anzugeben, die Umfangssegmente aus geschlossenen Zellen umfasst. Die Zellen sind durch jeweils wenigstens vier Stege begrenzt, die an Verbindungsstellen miteinander gekoppelt sind. Im expandierten Zustand bilden die Verbindungsstellen eine rautenförmige Grundform der Zelle. Von den mindestens vier Stegen sind zwei benachbarte Stege mit wenigstens zwei, insbesondere stegförmigen, Verbindungselementen verbunden, die miteinander gekoppelt sind. Die Verbindungselemente sind unterschiedlich flexibel derart, dass der Steg, der mit dem Verbindungselement mit niedrigerer Flexibilität verbunden ist, beim Übergang der Gitterstruktur vom expandierten Zustand in den komprimierten Zustand, stärker verformbar ist als der Steg, der mit dem Verbindungselement mit höherer Flexibilität verbunden ist. Dabei sind die, insbesondere alle, Zellen eines Umfangssegments gleichartig ausgebildet, so dass die gesamte Gitterstruktur beim Übergang vom expandierten Zustand in den komprimierten Zustand zumindest abschnittsweise tordiert. Die vorgenannten Merkmale und Eigenschaften der erfindungsgemäßen medizinischen Vorrichtung werden ebenso beim Übergang der Gitterstruktur vom komprimierten zum expandierten Zustand erreicht. Mit anderen Worten gilt das Vorgenannte nicht nur für die Komprimierung der Gitterstruktur, sondern auch für die Expansion.

Gemäß einem weiteren nebengeordneten Aspekt beruht die Erfindung auf dem Gedanken, ein Behandlungssystem mit der medizinischen Vorrichtung und einem Katheter anzugeben, wobei die medizinischen Vorrichtung ein Führungselement, insbesondere einen Führungsdraht, umfasst, das mit einem axialen Ende der Gitterstruktur fest, insbesondere drehfest, verbunden und längsverschieblich in dem Katheter angeordnet ist.

Der Übergang der Gitterstruktur vom expandierten Zustand in den komprimierten Zustand, also die Komprimierung der Gitterstruktur, erfolgt vorzugsweise bei der Herstellung des Behandlungssystems, wobei die Gitterstruktur vom expandierten Herstellzustand radial komprimiert wird, um die komprimierte Gitterstruktur in den Katheter einzubringen. Durch die Komprimierung der Gitterstruktur werden die Stege der einzelnen Zellen verformt. Dabei sind die Flexibilitätseigenschaften bzw. allgemein die Flexibilität der, insbesondere der beiden, Verbindungselemente unterschiedlich eingestellt, so dass sich die Verbindungselemente bei der Komprimierung der Gitterstruktur unterschiedlich flexibel verhalten. Insbesondere sind die Flexibilitätseigenschaften der beiden Verbindungselemente einer Zelle derart eingestellt, dass sich die gesamte Gitterstruktur bei der Komprimierung verdreht bzw. tordiert. Dieser Effekt tritt auch bei der Expansion der Gitterstruktur, also beim Übergang der Gitterstruktur vom komprimierten in den expandierten Zustand, auf.

Die Expansion der Gitterstruktur wird vorzugsweise beim Entlassen der komprimierten Gitterstruktur aus dem Katheter bewirkt. Die Expansion kann selbsttätig durch selbstexpandierbare Eigenschaften der Gitterstruktur erfolgen. Durch Wegfall des äußeren Komprimierungszwangs, der durch den Katheter auf die Gitterstruktur ausgeübt wird, werden die Radialkräfte der Gitterstruktur frei, so dass sich die Gitterstruktur in radialer Richtung aufweitet. Dabei bewirken die unterschiedlichen Flexibilitätseigerischaften der beiden Verbindungselemente ein unterschiedliches Verhalten der einzelnen Stege bei der Expansion. Insbesondere sind die Flexibilitätseigenschaften derart eingestellt, dass sich die gesamte Gitterstruktur bei der Expansion verdreht bzw. um die Längsachse tordiert.

Bei der Expansion der Gitterstruktur weisen die einzelnen Stege der Gitterstruktur eine Bewegungskomponente nicht nur in radiale Richtung, sondern zumindest segmentweise gleichzeitig in Umfangsrichtung auf. Dies gilt insbesondere für die Verbindungsstellen, die sich bei der Expansion der Gitterstruktur nicht nur in radialer Richtung von der Längsachse entfernen, sondern auch segmentweise eine Drehbewegung in Umfangsrichtung aufweisen. Die Drehbewegung kann segmentweise unterschiedlich stark ausgeprägt sein. Insbesondere kann die Drehbewegung derart ausgebildet sein, dass sich die in Längsrichtung beabstandeten Verbindungsstellen derart in Umfangsrichtung verdrehen, dass sich entlang der gesamten Gitterstruktur im Wesentlichen eine schraubenförmige Verdrehung bzw. Torsion einstellt.

Der Vorteil, der mit dem zuvor beschriebenen Expansionsmechanismus erreicht wird, besteht darin, dass sich die Gitterstruktur beim Aufweiten in einem Blutgefäß nicht nur radial, sondern auch in Umfangsrichtung in ein Blutgerinnsel einschneidet. Dadurch werden innerhalb des Blutgerinnsels Hinterschnitte ausgebildet, so dass die Verankerung der Gitterstruktur im Blutgerinnsel bzw. allgemein die Verbindung zwischen Gitterstruktur und Blutgerinnsel verbessert ist. Die Gefahr der Ablösung des Blutgerinnsels beim Entfernen aus einem Blutgefäß wird somit reduziert. Da die Stege der Gitterstruktur sich nicht nur in radialer Richtung, sondern auch gleichzeitig in Umfangsrichtung in das Blutgerinnsel einschneiden, wird der Weg der einzelnen Stege durch das Material des Blutgerinnsels verlängert. Dies trägt zur verbesserten Verankerung der Gitterstruktur im Blutgerinnsel bei. Insgesamt weist die erfindungsgemäße Vorrichtung also verbesserte Schneideigenschaften der Gitterstruktur auf, die zu einer verbesserten Arretierung der Gitterstruktur in einem Thrombus bzw. allgemein Blutgerinnsel führen.

Die unterschiedlichen Flexibilitäten der wenigstens zwei Verbindungselemente einer Zelle können auf verschiedene Art und Weise eingestellt werden. Insbesondere können die unterschiedlichen Flexibilitäten der einzelnen Verbindungselemente, insbesondere der Flexibilitätsunterschied zwischen dem Verbindungselement mit höherer Flexibilität und dem Verbindungselement mit niedrigerer Flexibilität, durch eine der im Folgenden erläuterten konstruktiven Ausführungsformen erreicht werden, wobei beliebige Kombinationen der genannten Ausführungsformen möglich sind.

Bei einer bevorzugten Ausführungsform wird die unterschiedliche Flexibilität der zwei Verbindungselemente einer Zelle dadurch erreicht, dass die Verbindungselemente jeweils unterschiedliche Längen aufweisen. Dabei weist das Verbindungselement, das eine größere Länge aufweist, eine größere Flexibilität auf. Das bedeutet, dass sich das längere Verbindungselement, also das Verbindungselement mit höherer Flexibilität, bei der Zustandsänderung der Gitterstruktur stärker verformt als das kürzere Verbindungselement, also das Verbindungselement mit kleinerer Flexibilität.

Alternativ oder zusätzlich kann das Verbindungselement mit höherer Flexibilität im Wesentlichen S-förmig ausgebildet sein. Das Verbindungselement mit niedrigerer Flexibilität ist hingegen geradlinig ausgebildet. Die S-förmige Ausbildung des Verbindungselements bewirkt, dass ein derartiges Verbindungselement länger ist als ein gleichwertiges geradliniges Verbindungselement. Somit wird die höhere Flexibilität einerseits durch die tatsächlich längere Elementlänge und andererseits durch die geometrische Form des Verbindungselements bewirkt. Die geometrische Form trägt insofern zur Flexibilität bei, als durch die S-förmige Krümmung eine stärkere Verformung, also eine stärkere Bewegung zwischen den Endpunkten des S-förmig gebogenen Verbindungselements beim Übergang der Gitterstruktur vom expandierten in den komprimierten Zustand und umgekehrt erreicht wird.

Das Verbindungselement mit höherer Flexibilität kann auch eine Elementbreite aufweisen, die kleiner als die Breite des Verbindungselements mit niedrigerer Flexibilität ist. Das Verbindungselement mit kleinerer Breite wirkt einer Verformung, die durch die Expansion oder Komprimierung der Gitterstruktur bewirkt wird, weniger stark entgegen, so dass das schmalere Verbindungselement insgesamt eine höhere Flexibilität aufweist. Das Verbindungselement mit kleinerer Breite lässt sich also stärker verformen, als das relativ steifere Verbindungselement mit größerer Elementbreite.

Eine weitere zusätzliche oder alternative Möglichkeit zur Einstellung der Flexibilität der Verbindungselemente besteht darin, dass das Verbindungselement mit höherer Flexibilität mit Biegestellen, insbesondere Verjüngungen oder Verbreiterngen oder Durchbrüchen, versehen ist. Beispielsweise kann das Verbindungselement mit höherer Flexibilität Einkerbungen, also lokal begrenzte Stellen, die sich gegenüber dem gesamten Verbindungselement durch eine Materialreduzierung auszeichnen, aufweisen. Die Biegestellen können dabei im Wesentlichen Sollbiegestellen oder Knickpunkte bilden, an denen das Verbindungselement vergleichsweise leicht gebogen werden kann. Die Biegestellen bewirken also eine höhere Flexibilität des Verbindungselements gegenüber einem Verbindungselement mit relativ niedrigerer Flexibilität. Die Verjüngungen bilden dabei Teilbereiche des Verbindungselements, die beispielsweise eine kleinere Breite als die restlichen Bereiche desselben Verbindungselements und insbesondere als das andere Verbindungselement mit niedrigerer Flexibilität aufweisen. Die Biegestellen können auch als Durchbrüche, beispielsweise als Fenster innerhalb eines Verbindungselements ausgebildet sein. Das Verbindungselement kann also lokal Öffnungen aufweisen, so dass die Materialstärke des Verbindungselements stellenweise reduziert ist. Dadurch wird erreicht, dass die Flexibilität des Verbindungselements erhöht ist. Gegenüber einem massiven Verbindungselement ohne derartige Durchbrüche, weist das Verbindungselement mit einem Durchbruch somit eine höhere Flexibilität auf.

Die Verbindungselemente können entweder mit zwei in Umfangsrichtung der Gitterstruktur benachbarten Stege oder mit zwei in Längsrichtung der Gitterstruktur benachbarten Stege gekoppelt sein.

Die Verbindungselemente können einen Schneidebereich aufweisen, der sich bei einer Rotation in Umfangsrichtung bewegt bzw. an dem Thrombus gleitet.

Zweckmäßigerweise ist bei der erfindungsgemäßen medizinischen Vorrichtung vorgesehen, dass die Stege an den Verbindungsstellen einstückig miteinander verbunden sind. Vorzugsweise sind die, insbesondere die beiden, Verbindungselemente an einer Kopplungsstelle insbesondere stoffschlüssig bzw. einstückig, miteinander verbunden. Eine unabhängige Relativbewegung der Stege untereinander und der Verbindungselemente relativ zueinander wird somit unterbunden bzw. die Relativbewegung erfolgt in Abhängigkeit von den jeweils miteinander verbundenen Elementen. Konkret erfolgt die Verformung der gesamten Gitterstruktur beim Übergang vom komprimierten Zustand in den expandierten Zustand und umgekehrt durch eine Verbiegung bzw. flexible Auslenkung der einzelnen Stege beziehungsweise Verbindungselemente. Ein Gleiten von Gitterelementen übereinander, wie beispielsweise bei Gittergeflechten vorgesehen, ist nicht vorgesehen.

Gemäß einer weiteren bevorzugten Ausführungsform der erfindungsgemäßen Vorrichtung sind die Verbindungselemente mit zwei benachbarten Stegen an zwei Kopplungsstellen verbunden. Die Verbindungselemente bilden mit den beiden Stegen, mit denen sie gekoppelt sind, eine Unterzelle innerhalb der übergeordneten Zelle bzw. Hauptzelle. Im expandierten Zustand wird eine viereckige Grundform, insbesondere eine konvexe viereckige Grundform, der Unterzelle gebildet, die durch die Kopplungsstellen, die Verbindungsstelle zwischen den benachbarten Stegen, und den Anschlusspunkt zwischen den Verbindungselementen definiert ist. Eine derartige Anordnung der Verbindungselemente in einer geschlossenen Zelle ist für die Verdrehfunktion bzw. Torsionsfunktion der Gitterstruktur besonders vorteilhaft.

Gemäß einer weiteren bevorzugten Ausführungsform der erfindungsgemäßen medizinischen Vorrichtung ist vorgesehen, dass die in Längsrichtung der Gitterstruktur gegenüberliegend angeordneten Verbindungsstellen einen unterschiedlichen Versetzungsgrad beim Übergang der Gitterstruktur vom expandierten in den komprimierten Zustand aufweisen. Zur Bestimmung des Versetzungsgrades wird zunächst ein Nullpunktzustand festgelegt. Vorzugsweise entspricht der Nullpunktzustand dem Zustand der Gitterstruktur, in dem die in Längsrichtung gegenüberliegend angeordneten Verbindungsstellen zueinander nicht versetzt sind, also in einer Linie fluchten, die sich parallel zur Längsachse der Gitterstruktur erstreckt. Bei einer Zustandsänderung der Gitterstruktur, beispielsweise bei einer Komprimierung oder Expansion ausgehend vom Nullpunktzustand, werden die in Längsrichtung benachbarten Verbindungsstellen aus der Nullpunktlage ausgelenkt. Die in Längsrichtung benachbart angeordneten bzw. gegenüberliegend angeordneten Verbindungsstellen versetzen sich zueinander.

Der Grad der Auslenkung bzw. des Versatzes kann unterschiedlich sein. Beispielsweise kann eine weiter proximal angeordnete Verbindungsstelle einer Zelle bei der Zustandsänderung der Gitterstruktur weiter aus der Nullpunktiage ausgelenkt bzw. versetzt werden, als eine weiter distal angeordnete Verbindungsstelle einer Zelle. Der Versetzungsgrad von in Umfangsrichtung benachbart angeordneten Verbindungsstellen eines Umfangssegments bleibt jedoch in jedem Zustand der Gitterstruktur gleich. Mit anderen Worten entspricht der Umfang eines Umfangssegments in jedem Zustand der Gitterstruktur einem Vielfachen des Abstands zwischen zwei in Umfangsrichtung benachbart angeordneten Verbindungsstellen des Umfangssegments.

Ein Umfangssegment ist aus einer in Umfangsrichtung der Gitterstruktur angeordneten Reihe angrenzender Zellen gebildet. Die Reihe bzw. das Umfangssegment ist in Umfangsrichtung geschlossen und begrenzt das Lumen der Gitterstruktur. Ein Umfangssegment ist aus wenigstens einer Reihe von Zellen, insbesondere aus mehreren Reihen gleichartiger, insbesondere denselben Zellen, gebildet.

Der Versetzungsgrad von in Längsrichtung gegenüberliegend angeordneten Verbindungsstellen kann von Umfangssegment zu Umfangssegment variieren. In Längsrichtung aufeinanderfolgende Umfangssegmente der Gitterstruktur können also unterschiedliche Versetzungsgrade aufweisen. Insbesondere können die Versetzungsgrade von in Längsrichtung benachbart angeordneten Umfahgssegmenten derart angepasst sein, dass sich die Rotations- bzw. Torsionsrichtung der Umfangssegmente bei der Zustandsänderung der Gitterstruktur gegenläufig einstellt. Ein Teil der Gitterstruktur kann also beim Übergang vom komprimierten Zustand in den expandierten Zustand und umgekehrt in positiver Drehrichtung tordieren und ein weiterer, in Längsrichtung der Gitterstruktur nachgeordneter Teil in negativer Drehrichtung. Insgesamt kann die Gitterstruktur mehrere Abschnitte aufweisen, die in unterschiedliche Richtungen tordieren. Dadurch kann die Verankerung eines Blutgerinnseln bzw. Thrombus mit der Gitterstruktur weiter verbessert werden.

Bei einer weiteren bevorzugten Ausführungsform der erfindungsgemäßen Vorrichtung spannen in Umfangsrichtung benachbart angeordnete Verbindungsstellen eines Umfangssegments eine gemeinsame Querschnittsebene der Gitterstruktur auf, die orthogonal zur Längsachse der Gitterstruktur angeordnet ist. Dabei bewegen sich die in Umfangsrichtung benachbart angeordneten Verbindungsstellen beim Übergang der Gitterstruktur vom expandierten Zustand in den komprimierten Zustand in der Querschnittsebene. Mit anderen Worten ist vorgesehen, dass in Umfangsrichtung beabstandet angeordnete Verbindungsstellen in jedem Zustand der Gitterstruktur zueinander eine Relativposition einnehmen, die durch eine orthogonale Ebene zur Längsachse der Gitterstruktur beschrieben ist. Während die in Längsrichtung beabstandet angeordneten Verbindungsstellen beim Übergang der Gitterstruktur vom komprimierten in den expandierten Zustand und umgekehrt, zueinander in Umfangsrichtung versetzt werden, erfolgt in Längsrichtung zwischen zwei in Umfangsrichtung benachbart angeordneten Verbindungsstellen kein Versatz. Vielmehr weisen die einzelnen Umfangssegmente ringförmig auf dem Umfang der Gitterstruktur angeordnete Verbindungsstellen auf, die zueinander fluchten. Die in Umfangsrichtung benachbarten Verbindungsstellen fluchten dabei in jedem Zustand der Gitterstruktur, insbesondere im komprimierten und im expandierten Zustand.

Die von den in Umfangsrichtung benachbart angeordneten Verbindungsstellen aufgespannte Querschnittsebene liegt in jedem Zustand der Expansion bzw. Komprimierung der Gitterstruktur vor, auch wenn sich die Querschnittsebene bei der Expansion oder der Komprimierung der Gitterstruktur insgesamt in Längsrichtung verschiebt. Eine derartige Verschiebung kann beispielweise durch das so genannte Foreshortening bewirkt sein, wobei sich die Gitterstruktur insgesamt verkürzt, wenn eine radiale Expansion erfolgt.

Durch die Anordnung der Verbindungsstellen auf einer gemeinsamen Querschnittsebene wird erreicht, dass sich die in Längsrichtung benachbart angeordneten Verbindungsstellen beim Übergang der Gitterstruktur vom komprimierten in den expandierten Zustand und umgekehrt zueinander versetzen. Insbesondere können sich die Verbindungsstellen, die in Längsrichtung der Gitterstruktur gegenüberliegend angeordnet und einer gemeinsamen Zelle zugeordnet sind, beim Übergang der Gitterstruktur vom expandierten Zustand in den komprimierten Zustand und umgekehrt gegenläufig in Umfangsrichtung der Gitterstruktur bewegen. Beispielsweise kann sich eine weiter proximal angeordnete Verbindungsstelle bei der Zustandsänderung der Gitterstruktur in Uhrzeigerrichtung bzw. in positiver Umlaufrichtung um die Längsachse der Gitterstruktur drehen, wogegen eine weiter distal angeordnete Verbindungsstelle bei derselben Zustandsänderung der Gitterstruktur eine Bewegung in Gegenuhrzeigerrichtung, also in negativer Umlaufrichtung, durchführen kann.

Die Erfindung gemäß Anspruch 1, wird im Folgenden anhand von Ausführungsbeispielen unter Bezugnahme auf die beigefügten, schematischen Zeichnungen näher erläutert. Darin zeigen
- Fig. 1: einen Ausschnitt aus einer Gitterstruktur mit drei Umfangssegmenten mit jeweils mehreren Zellen im expandierten Zustand;
- Fig. 2: der Ausschnitt der Gitterstruktur gemäß Fig. 1 im komprimierten Zustand;
- Fig. 3: eine Draufsicht auf eine Zelle der Gitterstruktur der medizinischen Vorrichtung gemäß einem Ausführungsbeispiels im expandierten Zustand;
- Fig. 4: die Zelle gemäß Fig. 3 im komprimierten Zustand;
- Fig. 5: einen Querschnitt durch ein Blutgefäß mit einem Thrombus und der darin angeordneten Gitterstruktur der beispielhaften medizinischen Vorrichtung im Gebrauch;
- Fig. 6: eine Draufsicht auf eine Zelle der Gitterstruktur der erfindungsgemäßen medizinischen Vorrichtung gemäß einem ersten Ausführungsbeispiel im expandierten Zustand;
- Fig. 7: eine Draufsicht auf eine Zelle der Gitterstruktur der erfindungsgemäßen medizinischen Vorrichtung gemäß einem zweiten Ausführungsbeispiel im expandierten Zustand;
- Fig. 8: einen Querschnitt durch ein Blutgefäß mit einem Thrombus und der darin angeordneten Gitterstruktur der erfindungsgemäßen medizinischen Vorrichtung mit einer Gitterstruktur mit Zellen gemäß Fig. 7;
- Fig. 9: einen Ausschnitt aus einer Gitterstruktur mit vier benachbart angeordneten Zellen im expandierten Zustand, wobei die Gitterstruktur Zellen gemäß Fig. 7 aufweist;
- Fig. 10: eine Draufsicht auf eine Zelle der Gitterstruktur der erfindungsgemäßen medizinischen Vorrichtung gemäß einem dritten Ausführungsbeispiel im expandierten Zustand; und
- Fig. 11: eine Draufsicht auf eine Zelle der Gitterstruktur der erfindungsgemäßen medizinischen Vorrichtung gemäß einem vierten Ausführungsbeispiel im expandierten Zustand.

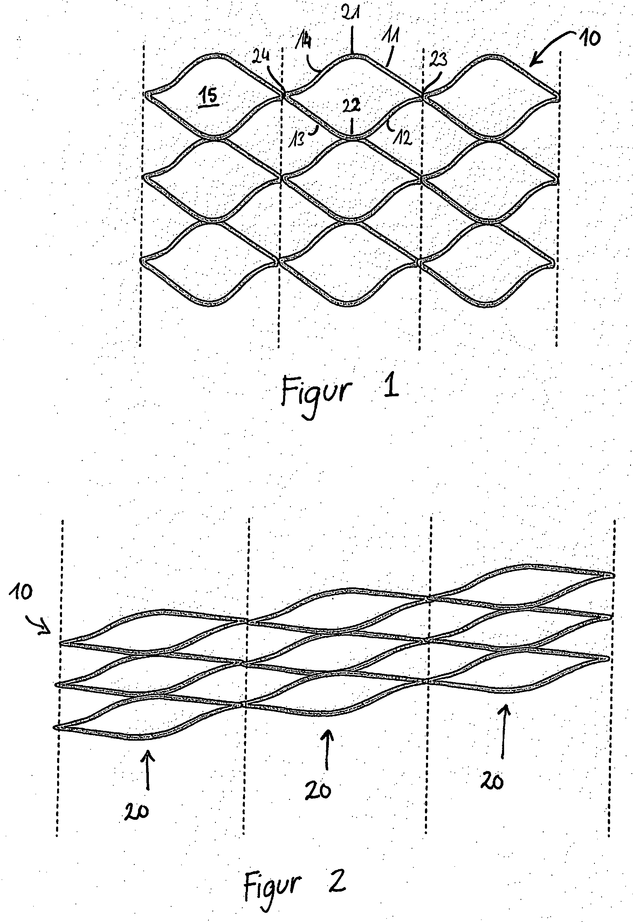

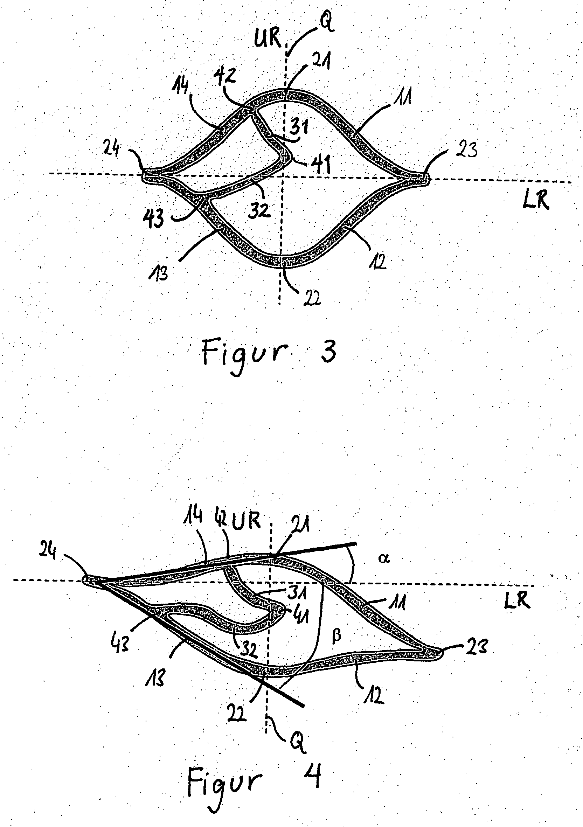

Fig. 1 zeigt einen Ausschnitt einer komprimierbaren und expandierbaren Gitterstruktur 10, die eine Vielzahl von Zellen 15 umfasst und dient zur Erläuterung des Rotationsprinzips, das den in den Fig. 3-6 gezeigten Ausführungsbeispielen zu Grunde liegt. Das nachfolgend beschriebene Rotationsprinzip wird im Zusammenhang mit den Ausführungsbeispielen gemäß Fig. 3-6 offenbart. Die Gitterstruktur 10 ist einstückig ausgebildet, insbesondere einstückig aus einem Vollmaterial durch Ausschneiden der Zellöffnungen hergestellt. Vorzugsweise ist die Gitterstruktur 10 durch Laserschneiden hergestellt bzw. bildet eine lasergeschnittene Gitterstruktur 10. Die Gitterstruktur 10 ist zumindest abschnittsweise kreiszylinderförmig ausgebildet. Das bedeutet, dass die Gitterstruktur 10 eine Wandungsebene bildet, die eine kreiszylindrische Form aufweist. Mit anderen Worten kann die Gitterstruktur 10 zumindest abschnittsweise röhrchenartig, insbesondere stentartig, ausgebildet sein. Zusätzlich kann die Gitterstruktur korbartig ausgebildet sein.

In diesem Zusammenhang wird darauf hingewiesen, dass sich die nachfolgende Detailbeschreibung der Gitterstruktur 10 auf den Herstellzustand, also den vollständig expandierten Zustand der Gitterstruktur 10 bezieht, soweit nichts anderes angegeben ist. Der Bezugspunkt für die medizinischen Richtungsarigaben "proximal" und "distal" ist der Anwender der medizinischen Vorrichtung bzw. des Behandlungssystems. Proximal angeordnete Elemente sind dem Anwender der Vorrichtung bzw. des Behandlungssystems also näher als distal angeordnete Elemente.

Der Ausschnitt der Gitterstruktur 10 gemäß Fig. 1 weist drei Umfangssegmente 20 auf, die jeweils durch drei geschlossene Zellen 15 gebildet sind. Dabei sind die dargestellten Umfangssegmente 20 gleichartig ausgebildet. Innerhalb eines Umfangssegments 20 sind die Zellen 15 jeweils gleichartig ausgebildet. Konkret sind die Zellen 15 gemäß Fig. 1 alle identisch. Eine Reihe von geschlossenen Zellen 15 erstreckt sich in Umfangsrichtung UR um die Längsachse der Gitterstruktur 10 und bildet einen Zellenring, der im Rahmen der Anmeldung als Umfangssegment 20 bezeichnet wird. Mehrere in axialer Richtung bzw. in Längsrichtung der Gitterstruktur 10 benachbarte und miteinander verbundene Umfangssegment 20 bilden die Gitterstruktur 10.

Die einzelnen Zellen sind, wie in Fig. 1 dargestellt, durch jeweils vier Stege 11, 12, 13, 14 begrenzt. Generell handelt es sich bei den Zellen im Zusammenhang mit der Erfindung und den Ausführungsbeispielen um geschlossene Zellen (closed cell design). Die Stege 11, 12, 13, 14 sind an vier Verbindungsstellen 21, 22, 23; 24 miteinander fest verbunden, insbesondere einstückig verbunden. Die Zelle 15 weist im Wesentlichen eine rautenförmige oder rhombusförmige Grundform auf. Das bedeutet, dass die Verbindungsstellen 21, 22, 23, 24 die Eckpunkte eines Rhombus bilden, wobei sich die Stege 11, 12, 13, 14 im Wesentlichen entlang der Seiten des Rhombus erstrecken und die Verbindungsstellen 21, 22, 23, 24 miteinander verbinden. Dabei kann die Form der Stege 11, 12, 13, 14 von der geraden Verbindungsstrecke zwischen zwei Verbindungsstellen 21, 22, 23, 24 abweichen. Vorzugsweise bilden die Stege 11, 12, 13, 14 entlang der Verbindungsstrecke eine gekrümmte, gebogene, insbesondere eine S-förmige Form. Die Grundform einer Raute bleibt erkennbar.

Die Zelle 15 umfasst einen ersten Steg 11, einen zweiten Steg 12, einen dritten Steg 13 und einen vierten Steg 14. Der erste Steg 11 erstreckt sich zwischen einer ersten Verbindungsstelle 21 und einer dritten Verbindungsstelle 23. Der zweite Steg 12 verbindet die dritte Verbindungsstelle 23 mit einer zweiten Verbindungsstelle 22. Zwischen der zweiten Verbindungsstelle 22 und einer vierten Verbindungsstelle 24 erstreckt sich der dritte Steg 13. Der vierte Steg 14 ist zwischen der ersten Verbindungsstelle 21 und der vierten Verbindungsstelle 24 angeordnet. Der erste Steg 11 ist bezüglich des vierten Stegs 14 in Umfangsrichtung UR der Gitterstruktur 10 benachbart angeordnet. Bezüglich des zweiten Stegs 12 ist der erste Steg 11 in Längsrichtung LR der Gitterstruktur 10 benachbart angeordnet. Der dritte Steg 13 und der vierte Steg 14 sind ebenfalls in Längsrichtung LR der Gitterstruktur 10 benachbart angeordnet. In Umfangsrichtung UR der Gitterstruktur 10 benachbart angeordnet sind auch der zweite Steg 12 und der dritte Steg 13.

In Fig. 1 ist gut erkennbar, dass jede Verbindungsstelle 21, 22, 23, 24 jeweils vier Stege 11, 12, 13, 14 miteinander verbindet. Dabei kann jede Verbindungsstelle 21, 22, 23, 24 jeweils vier Zellen 15 zugeordnet werden. Insbesondere bilden die in Umfangsrichtung gegenüberliegend angeordneten Verbindungsstellen 21, 22 jeweils eine erste Verbindungsstelle einer Zelle 15 und gleichzeitig eine zweite Verbindungsstelle 22 einer in Umfangsrichtung benachbarten Zelle 15. Die in Längsrichtung gegenüberliegend angeordneten Verbindungsstellen 23, 24 bilden jeweils eine dritte Verbindungsstelle 23 einer Zelle 15 und gleichzeitig eine vierte Verbindungsstelle 24 einer in Längsrichtung benachbart angeordneten Zelle 15.

Wie zuvor erläutert, weist die Zelle 15 im Wesentlichen eine rautenförmige Grundform auf. Die beiden Verbindungsgeraden zwischen der ersten Verbindungsstelle 21 und der zweiten Verbindungsstelle 22 sowie der dritten Vereindungsstelle 23 und der vierten Verbindungsstelle 24 halbieren sich also gegenseitig. Im Ruhezustand gemäß Fig. 1 halbiert die in Längsrichtung LR ausgerichtete Verbindungsgerade zwischen der dritten und vierten Verbindungsstelle 23, 24 die in Umfangsrichtung ausgerichtete Verbindungsgerade zwischen der ersten und zweiten Verbindungsstelle 21, 22. Die Verbindungsgerade zwischen der ersten und zweiten Verbindungsstelle 21, 22 halbiert auch die Verbindungsgerade zwischen der dritten und vierten Verbindungsstelle 23, 24. Die beiden Verbindungsgerade halbieren sich also gegenseitig.

Durch gestrichelte, senkrechte Linien sind in Fig. 1 mehrere Querschnittsebenen Q eingezeichnet, die durch jeweils in Umfangsrichtung benachbart angeordnete Verbindungsstellen 21, 22, 23, 24 aufgespannt sind. Dabei wird ersichtlich, dass die dritten und vierten Verbindungsstellen 23, 24 nicht nur in Längsrichtung der Gitterstruktur 10 gegenüberliegend, sondern auch in Umfangsrichtung UR der Gitterstruktur 10 gegenüberliegend angeordnet sind. Beim Übergang der Gitterstruktur vom expandierten Zustand in den komprimierten Zustand und umgekehrt gilt also auch für die dritten und vierten Verbindungsstellen 23, 24, dass diese in jedem Zustand der Gitterstruktur 10 auf einer gemeinsamen Querschnittsebene Q. angeordnet sind, wobei die Querschnittsebene Q nur im expandiertem Zustand die Längsachse der Gitterstruktur 10 unter einem rechten Winkel trifft.

Die in Längsrichtung LR der Gitterstruktur 10 benachbart angeordneten Verbindungsstellen 21, 22, 23, 24 fluchten im Ruhezustand gemeinsam mit der Längsachse der Gitterstruktur 10. Dabei wird deutlich, dass dies auch für die ersten und zweiten Verbindungsstellen 21, 22 gilt, die in einem Ruhezustand eine gemeinsam Nullpunktgerade umfassen können.

Fig. 2 zeigt den komprimierten Zustand des Gitterstrukturausschnitts gemäß Fig.. 1. Die Querschnittsebenen, die die in Umfangsrichtung benachbart angeordneten Verbindungsstellen 21, 22, 23, 24 umfassen, sind in Fig. 2 als durchgezogene senkrechte Linien dargestellt. Es ist zu erkennen, dass die in Umfangsrichtung benachbart angeordneten Verbindungsstellen 21, 22, 23, 24 auch im komprimierten Zustand eine gemeinsame Querschnittsebene Q aufspannen. Ferner ist Fig. 2 zu entnehmen, dass die in Längsrichtung der Gitterstruktur 10 benachbart angeordneten Verbindungsstellen 21, 22, 23, 24 bezüglich einer Nullpunktgeraden versetzt sind. Insbesondere sind die beiden Umfangssegment 20 zueinander versetzt bzw. in Umfangsrichtung UR der Gitterstruktur 10 verschoben.

Bei der Komprimierung der Gitterstruktur 10 verschieben sich die erste Verbindungsstelle 21 und die zweite Verbindungsstelle 22 in der Querschnittsebene Q und nähern sich dabei an. Der auf die Längsachse der Gitterstruktur 10 bezogene Radius der Kreislinie, die die Verbindungsstellen 21, 22 eines Umfangssegments verbindet, reduziert sich. Die in Längsrichtung LR der Gitterstruktur 10 gegenüberliegend angeordneten Verbindungsstellen 23, 24 werden zueinander versetzt bzw. ausgelenkt. Insbesondere rotiert die dritte Verbindungsstelle 23 gegenläufig zur vierten Verbindungsstelle 24 um die Längsachse der Gitterstruktur 10. In Fig. 2 ist gut zu erkennen, dass die dritte Verbindungsstelle 23 und die vierte Verbindungsstelle 24 gegeneinander verdreht bzw. versetzt sind.

Um die Funktion der medizinischen Vorrichtung, insbesondere die Rotation der gesamten Gitterstruktur 10, zu erreichen, weist jedes Umfangssegment 20 gleichartig ausgebildete Zellen 15 auf. Die Zellenform wiederholt sich also innerhalb eines Umfangssegments 20. Die einzelnen Zellen 15 eines Umfangssegments 20 sind dabei in den in Umfangsrichtung UR gegenüberliegend angeordneten Verbindungsstellen 21, 22 miteinander fest verbunden, insbesondere einstückig verbunden.

Fig. 3 zeigt eine freigeschnittene Zelle 15 einer Gitterstruktur 10 der medizinischen Vorrichtung gemäß einem bevorzugten Ausführungsbeispiel. Die Stege 11, 12, 13, 14 der Zelle 15 weisen die gleiche Flexibilität auf. Ferner weist die Zelle 15 zwei miteinander gekoppelte Verbindungselemente 31, 32 auf, die jeweils mit einem von zwei benachbarten Stegen 11, 14 verbunden sind.

Die Verbindungselemente 31, 32 können einerseits, wie bei den Ausführungsbeispielen gemäß Fig. 3 und 4 vorgesehen ist, mit zwei in Umfangsrichtung benachbarten und miteinander verbunden Stegen 11, 12, 13, 14 verbunden sein. Konkret sind die Verbindungselemente 31, 32 bei dem Ausführungsbeispiel gemäß Fig. 3 und 4 mit dem dritten Steg 13 und dem vierten Steg 14 gekoppelt. Dabei ist das erste Verbindungselement 31 mit dem vierten Steg 14 und das zweite Verbindungselement 32 mit dem dritten Steg 13 verbunden. Die Verbindung zwischen den Verbindungselementen 31, 32 und den Stegen 11, 12, 13, 14 erfolgt außerhalb der Verbindungsstellen 21, 22, 23, 24, insbesondere zwischen den Verbindungsstellen 21, 22, 23, 24 und beabstandet von den Verbindungsstellen 21, 22, 23, 24.

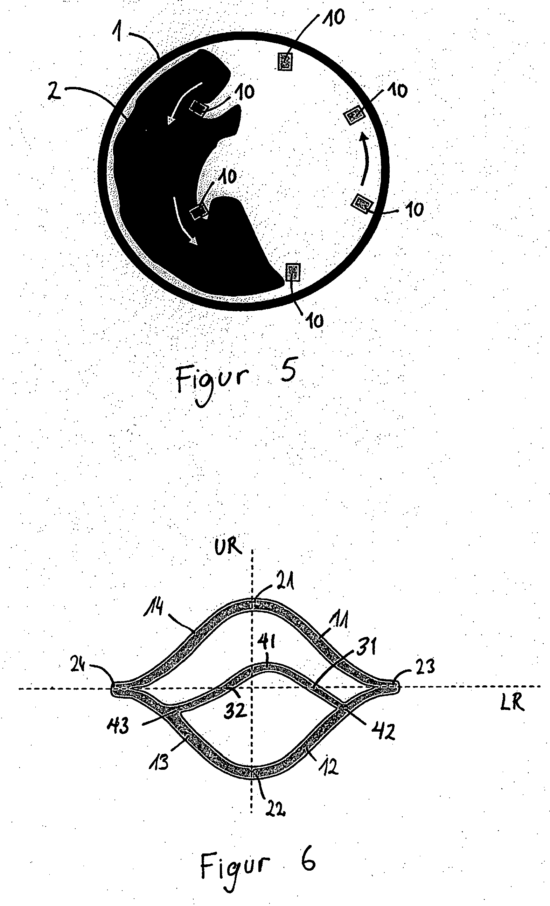

Alternativ kann vorgesehen sein, dass die Verbindungselemente 31, 32 mit zwei in Längsrichtung der Gitterstruktur 10 benachbarten Stegen 11, 12, 13, 14 gekoppelt sind. Grundsätzlich sind die Verbindungselemente 31, 32 mit Stegen der Zelle 15 verbunden, die in einer Verbindungsstelle 21, 22, 23, 24 miteinander gekoppelt und unmittelbar zueinander benachbart sind. Bei den Ausführungsbeispielen gemäß Fig. 6-11 sind die Verbindungselemente 31, 32 mit zwei unmittelbar in Längsrichtung der Gitterstruktur 10 benachbarten Stegen 11, 12, 13, 14 der Zelle 15 verbunden.

Gemäß dem ersten, zweiten, dritten und vierten Ausführungsbeispiel (Fig. 6-11) ersten bilden die Verbindungselemente 31, 32 im Wesentlichen Abschnitte eines Zwischenstegs 30, der mit den beiden Stegen 11, 12, 13, 14, die er verbindet, eine Unterzelle 16 formt. Die Verbindungselemente 31, 32 sind zur Bildung des Zwischenstegs vorzugsweise stoffschlüssig bzw. einstückig miteinander verbunden.

Die als Verbindungselemente 31, 32 bezeichneten Abschnitte des Zwischenstegs 30 sind in einer Spitze 33 zusammengeführt. Die Spitze 33 zeigt bzw. weist in Umfangsrichtung der Gitterstruktur 10. Mit anderen Worten weist der Zwischensteg 30 in den dargestellten Ausführungsbeispielen eine Krümmung auf, die in Umfangsrichtung der Gitterstruktur 10 gewölbt ist. Der höchste Punkt der Krümmung, also die Spitze 33, ist somit in Umfangsrichtung der Gitterstruktur 10 versetzt gegenüber den zweiten Kopplungsstellen 42, 43 angeordnet in denen die Verbindungselemente 31, 32 mit den Stegen 11, 12, 13, 14 verbunden sind.

Konkret ist vorgesehen, dass die Unterzelle 16 analog zur übergeordneten Zelle 15 durch vier Stege begrenzt ist. Zwei der Stege der Unterzelle 16 sind durch Teitabschnitte der Stege 11, 12, 13, 14 der übergeordneten Zelle 15 bzw. Hauptzelle gebildet. Zwei weitere Stege der Unterzelle 16 sind durch die Verbindungselemente 31, 32 gebildet. Entsprechend weist die Unterzelle 16 vier Verbindungsstellen auf, wobei zwei Verbindungsstellen durch die zweiten Kopplungsstellen 42, 43 der Verbindungselemente 31, 32 mit den Stegen 11, 12, 13, 14 der übergeordneten Zelle 15 gebildet sind. Eine weitere Verbindungsstelle der Unterzelle 16 entspricht der Verbindungsstelle 21, 22, 23, 24 zwischen den beiden Stegen 11, 12, 13, 14 der übergeordneten Zelle 15, deren Teilabschnitte Stege der Unterzelle 16 bilden. Die vierte Verbindungsstelle der Unterzelle 16 ist durch die erste Kopplungsstelle 41 zwischen den beiden Verbindungselementen 31, 32 gebildet.

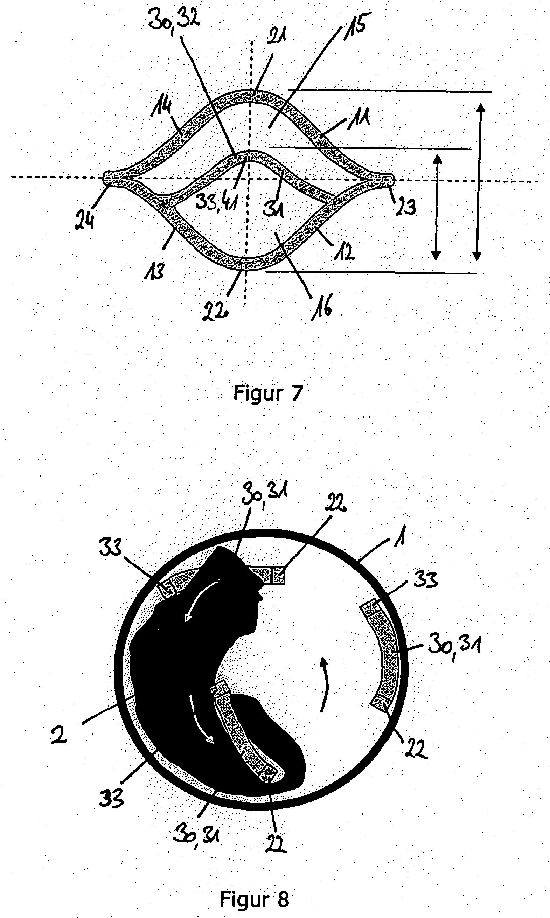

Der Aufbau der Unterzelle 16 entspricht also im Wesentlichen dem Aufbau der übergeordneten Zelle 15. Im Unterschied zu der übergeordneten Zelle 15 weist die Unterzelle 16 jedoch eine Verbindungsstelle, nämlich die erste Kopplungsstelle 41, auf, die frei angeordnet ist. Bei der übergeordneten, Zelle 15 ist, mit Ausnahme der axialen Enden der Gitterstruktur 10, grundsätzlich vorgesehen, dass alle Verbindungsstellen 21, 22, 23, 24 einer Zelle 15 gleichzeitig eine Verbindungsstelle 21, 22, 23, 24 einer benachbarten Zelle 15 bilden. Bei den Unterzellen ist jedoch eine Verbindungsstelle, nämlich die Kopplungsstelle 41, ausschließlich einer einzigen Unterzelle 16 zugeordnet und weist keine Verbindung zu einer weiteren Unterzelle 16 oder einer übergeordneten Zelle 15 auf. Auf diese Weise wird ermöglicht, dass sich die erste Kopplungsstelle 41, die gleichzeitig die Spitze 33 des Zwischenstegs 30 bildet, im expandierten Zustand der Gitterstruktur 10 aus der Umfangsebene der Gitterstruktur 10 erhebt und somit gut in ein Blutgerinnsel einschneidet. Dies ist in Fig. 8 gut erkennbar.

Insbesondere zeigt das Ausführungsbeispiel gemäß Fig. 8, dass sich im expandierten Zustand der Gitterstruktur die Spitze 33 des Zwischenstegs 30 auf einer größeren Querschnittsumfangsebene angeordnet ist als die Verbindungsstelle 21, 22, 23, 24 der übergeordneten Zelle 15. Somit ist zwischen der Umfangsebene, in der die übergeordnete Zelle 15 angeordnet ist, und der Umfangsebene, in der die Spitze 33 angeordnet ist, eine Lücke bzw. ein Spalt gebildet, in den das Blutgerinnsel 2 eindringen kann. Die Gitterstruktur 10 wird dadurch in dem Blutgerinnsel 2 verankert.

Grundsätzlich ist Erfindung, wobei ein im Wesentlichen in Längsrichtung der Gitterstruktur ausgerichteter Zwischensteg 30 vorgesehen ist, unabhängig von einer selbsttätigen Rotation bzw. Torsion der Gitterstruktur 10. In Fig. 7 ist beispielsweise eine Zellenkonstruktion dargestellt, bei der sowohl die Stege 11, 12, 13, 14 der übergeordneten Zelle 15, als auch die Verbindungselemente 31, 32 im Wesentlichen dieselbe Flexibilität aufweisen. Zwar können die Verbindungselemente 31, 32 eine andere Flexibilität aufweisen als die Stege 11, 12, 13, 14 der Zelle 15. Dieser Flexibilitätsunterschied führt jedoch nicht dazu, dass die Gitterstruktur bei der Expansion tordiert.

Bei dem Ausführungsbeispiel gemäß Fig. 7 ist der Zwischensteg 30 im Wesentlichen symmetrisch ausgebildet. Das bedeutet, dass die Verbindungselemente 31, 32, die im Wesentlichen zwei Halbabschnitte des Zwischenstegs 30 bilden, im Wesentlichen die gleiche, spiegelverkehrte Form aufweisen. Im Rahmen der Anmeldung wird dies als symmetrische Unterzelle 16 bezeichnet. Ein wesentliches Merkmal der symmetrischen Unterzelle 16 besteht darin, dass die Kopplungsstelle 41 bzw. die Spitze 33 des Zwischenstegs 30 auf derselben Querschnittsebene angeordnet ist, wie die erste Verbindungsstelle 21 der übergeordneten Zelle 15. Auf derselben Querschnittsebene ist im Übrigen die zweite Verbindungsstelle 22 der Zelle 15 angeordnet.

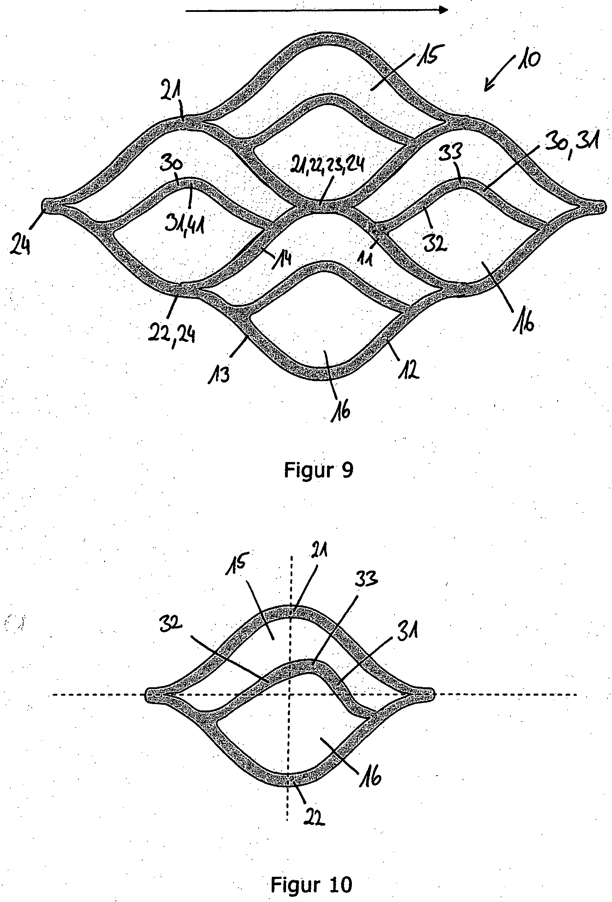

In Fig. 9 ist ein Ausschnitt aus einer Gitterstruktur 10 dargestellt, die mehrere, unmittelbar benachbarte Zellen aufweist, die nach dem Ausführungsbeispiel gemäß Fig. 7 ausgebildet sind. Durch einen Pfeil ist in Fig. 9 ferner die Längsachse der Gitterstruktur 10 angezeigt.

In der Querschnittsdarstellung gemäß Fig. 8 ist gut erkennbar, wie das Eingreifen der Gitterstruktur 10 in ein Blutgerinnsel 2 erfolgt, wenn die Gitterstruktur nach dem Ausführungsbeispiel gemäß Fig. 7 gestaltet ist bzw. Zellen gemäß Fig. 7 aufweist. Durch Pfeile ist in Fig. 8 eine Rotation der Gitterstruktur 10 angedeutet, wobei die Rotation vorzugsweise manuell erfolgt. Mit anderen Worten wird die Gitterstruktur 10 im expandierten Zustand rotiert, um zu erreichen, dass die in Umfangsrichtung orientierte Spitze 33 der Unterzelle 16 in das Blutgerinnsel 2 eingreift.

Die Spitze 33 der Unterzelle 16 kann bei der Herstellung der Gitterstruktur 10 bereits radial nach außen vorgeformt sein, wodurch das Eingreifen der Spitze 33 in ein Blutgerinnsel 2 begünstigt wird. Es ist jedoch auch möglich, dass die Spitze 33 bei der Herstellung der Gitterstruktur 10 in derselben Umfangsebene angeordnet ist, wie die übergeordnete Zelle 15. Weil die Teilabschnitte bzw. die Vereindungselemente 31, 32 des Zwischenstegs 30 kürzer als die parallel angeordneten Stege 11, 14 der übergeordneten Zelle 15 sind, wird die Spitze 33 bei einer Expansion der Gitterstruktur 10 dennoch radial nach außen gelenkt.

Es ist auch möglich, dass die radial nach außen vorstehende Spitze 33 der Unterzelle 16 zusätzlich mit einer selbstrotierenden bzw. selbsttordierenden Funktion der Gitterstruktur 10 kombiniert ist. Ein derartiges Ausführungsbeispiel zeigt Fig. 10. Darin ist erkennbar, dass die Unterzelle 16 eine asymmetrische Form aufweist. Das bedeutet, dass die Spitze 33 nicht in derselben Querschnittsebene angeordnet ist, wie die in Umfangsrichtung benachbarten Verbindungsstellen 21, 22 der übergeordneten Zellen 15. Vielmehr ist vorgesehen, dass der Zwischensteg 30 durch zwei Verbindungselemente 31, 32 gebildet ist, die unterschiedliche Längen aufweisen. Konkret ist das erste Verbindungselement 31 bei dem Ausführungsbeispiel gemäß Fig. 10 kürzer als das zweite Verbindungselement 32. Alternativ oder zusätzlich können die Verbindungselemente 31, 32 durch Biegestellen und/oder unterschiedliche Materialien verschiedene Flexibilitäten aufweisen. Auf die verschiedenen Möglichkeiten zur Einstellung unterschiedlicher Flexibilitäten wird später näher eingegangen.

Die Rotation der Gitterstruktur 10 erfolgt vorzugsweise in Richtung der Spitze 33 bzw. in derselben Richtung, in die die Spitze 33 zeigt. Dadurch wird erreicht, dass sich die Spitze 33 in das Blutgerinnsel 2 einschneidet bzw. sogar bei Kontakt mit dem Blutgerinnsel 2 weiter radial aufgestellt wird. Alternativ kann vorgesehen sein, dass die Rotation der Gitterstruktur 10 gegenläufig zur Ausrichtung der Spitze 33 erfolgt, um das Eindringen der Spitze 33 in das Blutgerinnsel 2 schonend zu gestalten oder das Blutgerinnsel 2 lediglich mit der Spitze 33 zu streifen.

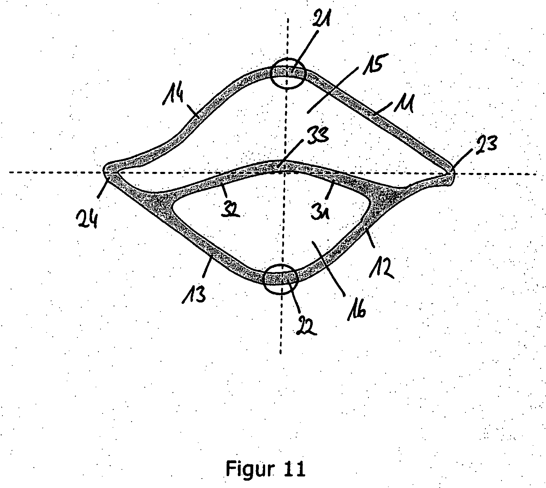

Die Rotation der Gitterstruktur 10 kann nicht nur durch eine asymmetrische Gestaltung der Unterzelle 16, sondern auch eine asymmetrische Gestaltung der Hauptzelle bzw. übergeordneten Zelle 15 erfolgen, wie Fig. 11 zeigt. Bei dem Ausführungsbeispiel gemäß Fig. 11 ist die Unterzelle 16 nahezu symmetrisch gestaltet. Die leichte Abweichung von einer vollständig symmetrischen Gestaltung wird ausschließlich durch die Asymmetrie der übergeordneten Zelle 15 bewirkt, so dass im Rahmen der Anmeldung die Unterzelle 16 gemäß Fig. 11 als symmetrisch bezeichnet wird. Die übergeordnete Zelle 15 weist hingegen jeweils zwei parallele Stege 11, 12, 13, 14 auf, die unterschiedliche Flexibilitäten aufweisen. Dazu ist vorgesehen, dass der zweite und vierte Steg 12, 14 eine S-förmig gekrümmte Form aufweist und der erste und dritte Steg 11, 13 im Wesentlichen geradlinig verlaufen. Dadurch wird bewirkt, dass die zweiten und vierten Verbindungsstellen 22, 24 bei der Expansion der Gitterstruktur 10 zueinander versetzt werden, wodurch eine Rotation bzw. Torsion der Gitterstruktur 10 ausgelöst wird. Die übergeordnete, asymmetrische Zelle 15 bewirkt also die Rotation oder Gitterstruktur 10, wogegen die im Wesentlichen symmetrische Unterzelle 16 das Eingreifen der Gitterstruktur 10 in das Blutgerinnsel 2 begünstigt. Eine Kombination einer asymmetrischen Unterzelle 16 mit einer asymmetrischen übergeordneten Zelle 15 ist ebenfalls möglich.

Die Unterzelle 16 weist vorzugsweise eine Breite auf, die wenigstens dem 0,2-fachen, insbesondere wenigstens dem 0,4-fachen, insbesondere wenigstens dem 0,6-fachen, insbesondere wenigstens dem 0,8-fachen, der Breite der übergeordneten Zelle 15 entspricht. Die Breite der Zelle 15 bzw. der Unterzelle 16 wird jeweils in Umfangsrichtung der vollständig expandierten Gitterstruktur 10 ermittelt und ist in Fig. 7 entsprechend dargestellt. Dabei ist nicht ausgeschlossen, dass sich in bestimmten Zustandsphasen der Gitterstruktur 10 beim Übergang vom komprimierten Zustand in den expandierten Zustand die Unterzelle 16 und die Zelle 15 überlappen. Konkret kann der Zwischensteg 30 bzw. die Spitze 33 beim Übergang der Gitterstruktur 10 vom radial komprimierten in den radial expandierten Zustand den ersten und/oder vierten Steg 11, 14 der übergeordneten Zelle 15 überlappen.

Bei einer medizinischen Vorrichtung, die eine Gitterstruktur 10 aus Zellen 15 und Unterzellen 16 aufweist, kann die Herstellung der Gitterstruktur 10 entweder durch Laserschneiden der Gitterstruktur 10 aus einem Rohr erfolgen, so dass die Stege 11, 12, 13, 14 der Zelle 15 und der Zwischensteg 30 einteilig aus einem Vollmaterial hergestellt sind.

Alternativ kann vorgesehen sein, dass die Gitterstruktur 10 durch zwei Untergitterstrukturen gebildet ist, wobei eine erste Untergitterstruktur aus Zellen 15, die später die übergeordneten Zellen 15 bilden, gebildet ist und eine zweite Untergitterstruktur, die Unterzellen 16 umfasst. Die beiden Untergitterstrukturen werden im Herstellungsprozess übereinander angeordnet und anschließend fest miteinander verbunden, beispielsweise verschweißt. Die übergeordneten Zellen 15 sind dann in einer anderen Umfangsebene angeordnet als die Unterzellen 16. Vorzugsweise wird die zweite Untergitterstruktur, die die Unterzellen 16 aufweist, radial außerhalb bzw. oberhalb der ersten Untergitterstruktur angeordnet. Dadurch wird erreicht, dass die Unterzellen 16 auf einer äußeren Umfangsebene der Gitterstruktur 10 und die übergeordneten Zellen 15 auf einer inneren Umfangsebene der Gitterstruktur 10 angeordnet sind.

Bei den Ausführungsbeispielen gemäß Fig. 1 bis 6 und 10 bis 11 sind die Verbindungselemente 31, 32 unterschiedlich flexibel derart, dass der Steg 11, der mit dem Verbindungselement 31 mit niedrigerer Flexibilität verbunden ist, beim Übergang der Gitterstruktur 10 vom expandierten Zustand in den komprimierten Zustand, stärker verformbar ist als der Steg 14, der mit dem Verbindungselement 32 mit höherer Flexibilität verbunden ist. Insbesondere ist vorgesehen, dass die zwei Verbindungselemente 31, 32, die eine gemeinsame Verbindungsstelle 41 aufweisen, unterschiedlich flexibel sind.

Dabei ist die Flexibilität der Verbindungselemente 31, 32 derart eingestellt dass ein Verbindungselement 31 eine höhere Flexibilität als das andere Verbindungselement 32 aufweist. Bei dem Ausführungsbeispiel gemäß Fig. 3 ist insbesondere vorgesehen, dass das zweite Verbindungselement 32 eine höhere Flexibilität aufweist als das erste Verbindungselement 31.

Als Flexibilität wird im Rahmen der Anmeldung das Vermögen der beiden Verbindungselemente 31, 32 bezeichnet, sich beim Übergang vom expandierten Zustand in den komprimierten Zustand zu verformen bzw. zu biegen. Der Grad der Auslenkung des angrenzenden Stegs 11, 12, 13, 14 aus dem Ruhezustand bei Einwirkung einer vorbestimmten, Kraft bestimmt die Höhe der Flexibilität des Verbindungselements 31, 32. Eine höhere Flexibilität des Verbindungselements 31, 32 bedeutet eine größere Auslenkung eines Stegs 11, 12, 13, 14. Mit anderen Worten ist das erste Verbindungselement 31 flexibler als das zweite Verbindungselement 32, wenn sich der angrenzende Steg 11 des ersten Verbindungselements 31 unter Einwirkung derselben Kraft stärker verformt bzw. stärker ausgelenkt wird als der Steg 14 der mit dem zweiten Verbindungselement 32 verbunden ist.

Die Flexibilität des ersten Verbindungselements 31 gegenüber dem zweiten Verbindungselement 32 kann auf unterschiedliche Weise eingestellt werden. Einerseits kann die Flexibilität der Verbindungselemente 31, 32 dadurch erhöht werden, dass das Verbindungselement 31, 32 nicht geradlinig, sondern gekrümmt bzw. S-förmig ausgebildet ist.

Eine weitere Möglichkeit, die Flexibilität der Verbindungselemente 31, 32 einzustellen, besteht darin, die Länge des Verbindungselementes 31, 32 zu variieren. Diese Variante der Erhöhung der Flexibilität ist bei dem Ausführungsbeispiel gemäß Fig. 3 und 4 bei den Verbindungselementen 31, 32 verwirklicht. Das erste Verbindungselement 31 und das zweite Verbindungselemente 32 weisen unterschiedliche Längen auf. Das erste Verbindungselement 31 ist kürzer als das zweite Verbindungselement 32, und weist daher eine niedrigere Flexibilität auf. Ein längeres Verbindungselement 31, 32 weist eine höhere Flexibilität als ein kürzeres Verbindungselement 31, 32 auf.

Dies ist bei dem Ausführungsbeispiel gemäß Fig. 6 ebenfalls verwirklicht. Insbesondere ist das zweite Verbindungselement 32, das im Wesentlichen die gleiche Form wie das erste Verbindungselement 31 aufweist, länger als das erste Verbindungselement 31. Das zweite Verbindungselement 32 weist daher eine höhere Flexibilität als das erste Verbindungselement 31 auf.

Eine weitere Variante zur Änderung der Flexibilität der Verbindungselemente 31, 32 ist eine Variation der Breite der Verbindungselemente 31, 32. Im Allgemeinen weist ein Verbindungselement mit relativ kleinerer Breite eine höhere Flexibilität als ein Verbindungselement mit relativ größerer Breite auf bezogen auf das jeweils andere Verbindungselement. Durch Variation der Breite des Verbindungselements 31, 32 kann also die Flexibilität eingestellt werden. Ferner kann die Flexibilität der Verbindungselemente 31, 32 durch den Einsatz von Biegestellen eingestellt werden. Derartige Biegestellen können an beliebiger Stelle eines Verbindungselements eingebracht sein und beispielsweise Verjüngungen, also lokale Änderungen der Breite des Verbindungselements, umfassen.

Es ist auch denkbar, die Biegestellen durch Verbreiterungen des Verbindungselements 31, 32 zu realisieren. Ferner können die Biegestellen als Fenster bzw. Durchbrüche gebildet sein. Beispielsweise kann ein Verbindungselement 31, 32 gezielt mit Bohrungen bzw. Löchern versehen sein, so dass eine Biegestelle bzw. eine Knickstelle bereitgestellt wird. Durch eine gezielte stellenweise Materialreduktion können also Biegestellen bereitgestellt werden, die der Verformung des Verbindurigselements 31, 32 beim Übergang der Gitterstruktur, vom expandierten Zustand in den komprimierten Zustand frühzeitig nachgeben, so dass sich das Verbindungselement 31, 32 insgesamt stärker verformt. Das Verbindungselement 31, 32 weist somit eine höhere Flexibilität als ein anderes Verbindungselement 31, 32 ohne Biegestellen auf.

Die Zelle 15 gemäß Fig. 3 liegt im expandierten Zustand der Gitterstruktur 10 vor. Dabei ist zu erkennen, dass die erste Verbindungsstelle 21 und die zweite Verbindungsstelle 22 eine Verbindungslinie aufweisen, die sich quer durch die Zelle 15 erstreckt und in Umfangsrichtung UR der Gitterstruktur 10 verläuft. Konkret bildet die Verbindungslinie zwischen den in Umfangsrichtung gegenüberliegend angeordneten Verbindungsstellen 21, 22 eine Kreislinie, die sich um den Umfang der Gitterstruktur 10 erstreckt. Insgesamt sind die in Umfangsrichtung UR gegenüberliegend angeordneten Verbindungsstellen 21, 22 eines Umfangssegments 20 der Gitterstruktur 10 in einer gemeinsamen Ebene Q angeordnet, die orthogonal auf der Längsachse der Gitterstruktur 10 steht. Die gemeinsame Querschnittsebene Q, die alle in Umfangsrichtung UR benachbart bzw. gegenüberliegend angeordneten Verbindungsstellen 21, 22 eines Umfangssegments 20 umfasst, ist in den Fig. 5 dargestellt.

In Fig. 3 ist ferner zu erkennen, dass die in Längsrichtung LR der Gitterstruktur 10 gegenüberliegend angeordneten Verbindungsstellen 23, 24, die durch die dritte und vierte Verbindungsstelle 23, 24 gebildet sind, auf einer Linie angeordnet sind, die sich in der Zeichnungsebene horizontal erstreckt und als gestrichelte Linie dargestellt ist. Die Verbindungslinie zwischen den dritten und vierten Verbindungsstellen 23, 24 erstreckt sich im expandierten Zustand der Gitterstruktur 10 gemäß Fig. 1 parallel zur Längsachse der Gitterstruktur 10. Die Verbindungslinie zwischen den dritten und vierten Verbindungsstellen 23, 24 steht also senkrecht auf der Querschnittsebene Q.

In Fig. 4 ist verdeutlicht, wie sich der unterschiedliche Versatz der Verbindungsstellen 23, 24, die in Längsrichtung der Gitterstruktur 10 gegenüberliegend angeordnet sind, bei der Zustandsänderung der Gitterstruktur 10 einstellt.

In Fig. 3 ist die Zelle 15 der Gitterstruktur 10 im Ruhezustand dargestellt. Insbesondere weist die Zelle 15 einen ersten Steg 11, einen zweiten Stege 12, einen dritten Steg 13 und einen vierten Steg 14, sowie ein erstes Verbindungselement 31 und ein zweites Verbindungselement 32 auf, wobei das zweite Verbindungselement 32 eine höhere Flexibilität als das erste Verbindungselement 31 aufweist. Im Ruhezustand sind die Verbindungsstellen 21, 22, 23, 24 zwischen den einzelnen Stegen 11, 12, 13, 14 derart ausgerichtet, dass sie eine rautenförmige Grundform der Zelle 15 aufspannen. Insbesondere sind die in Längsrichtung gegenüberliegend angeordneten Verbindungsstellen 23, 24 auf einer gemeinsamen Verbindungsgeraden angeordnet, die sich im Wesentlichen parallel zur Längsachse der Gitterstruktur 10 erstreckt und als Nullpunktgerade bezeichnet wird.

Bei der Komprimierung der Gitterstruktur 10 verschieben sich die erste Verbindungsstelle 21 und die zweite Verbindungsstelle 22 in der Querschnittsebene Q und nähern sich dabei an. Der auf die Längsachse der Gitterstruktur 10 bezogene Radius der Kreislinie, die die Verbindungsstellen 21, 22 eines Umfangssegments verbindet, reduziert sich. Aufgrund der unterschiedlichen Flexibilitäten der Verbindungselemente 31, 32 werden die in Längsrichtung LR der Gitterstruktur 10 gegenüberliegend angeordneten Verbindungsstellen 23, 24 zueinander versetzt bzw. ausgelenkt. Insbesondere bewirkt die unterschiedliche Flexibilität der Verbindungselemente 31, 32, dass die dritte Verbindungsstelle 23 gegenläufig zur vierten Verbindungsstelle 24 um die Längsachse der Gitterstruktur 10 rotiert. In Fig. 4 ist gut zu erkennen, dass die dritte Verbindungsstelle 23 und die vierte Verbindungsstelle 24 gegeneinander verdreht bzw. versetzt sind.

In Fig. 4 sind ferner die Winkel des ersten bzw. vierten Stegs 11, 14 durch fett gedruckte Linien eingezeichnet. Es ist zu erkennen, dass die Winkel α des ersten Stegs 11 bezüglich der in Längsrichtung LR verlaufenden Verbindungslinie sich vom Winkel β des vierten Stegs 14 bezüglich derselben Verbindungslinie unterscheidet. Daraus ist zu erkennen, dass die Flexibilitäten der Verbindungselemente 31, 32 derart unterschiedlich sind, dass der Versetzungsgrad der in Längsrichtung gegenüberliegend angeordneten Verbindungsstellen 23, 24 unterschiedlich ist. Insbesondere wird bei dem Ausführungsbeispiel gemäß Fig. 3 und 4 die vierte Verbindungsstelle 24 beim Übergang der Gitterstruktur vom expandierten Zustand in den komprimierten Zustand weiter aus der ursprünglichen Lage gemäß Fig. 3 ausgelenkt als die dritte Verbindungsstelle 23. Konkret werden die dritte Verbindungsstelle 23 und die vierte Verbindungsstelle 24 bezüglich der Nullpunktgeraden, die parallel zur Längsachse der Gitterstruktur 10 verläuft, ausgelenkt, wie in Fig. 4 dargestellt. Dabei weist die vierte Verbindungsstelle 24 einen höheren Auslenkgrad β als die dritte Verbindungsstelle 23 auf, die einen relativ kleineren Auslenkgrad α aufweist. Der Abstand der vierten Verbindungsstelle 24 von der Nullpunktsgeraden ist im komprimierten Zustand der Gitterstruktur 10 also größer als der Abstand der dritten Verbindungsstelle 23 von der Nullpunktsgeraden.

Die erste Verbindungsstelle 21 und die zweite Verbindungsstelle 22 sind in beiden Zuständen, also im expandierten Zustand gemäß Fig. 3 und im komprimierten Zustand gemäß Fig. 4, auf einer gemeinsamen Querschnittsebene Q angeordnet. Zwar kann sich die Querschnittsebene Q insgesamt in Längsrichtung der Gitterstruktur 10 bei der Zustandsänderung verschieben. Die einzelnen in Umfangsrichtung benachbart angeordneten Verbindungsstellen 21, 22 bleiben jedoch in jedem Zustand der Gitterstruktur 10 auf der gemeinsamen, ggf. verschobenen, Querschnittsebene Q.

Die Funktion der Gitterstruktur 10 der medizinischen Vorrichtung besteht darin, dass bei der Komprimierung und vor allem bei der Expansion zumindest abschnittsweise eine Torsion der Gitterstruktur 10 erreicht wird. Da sich die in Längsrichtung benachbart bzw. gegenüberliegend angeordneten Verbindungsstellen 23, 24 bei der Zustandsänderung der Gitterstruktur 10, insbesondere beim Übergang vom expandierten Zustand zum komprimierten Zustand, zueinander versetzen bzw. unterschiedlich schnell um die Längsachse der Gitterstruktur 10 rotieren, werden die nachfolgenden Umfangssegmente 20 insgesamt verdreht. Die Verdrehung bzw. Torsion erfolgt auch beim Übergang der Gitterstruktur 10 vom komprimierten in den expandierten Zustand, wobei die in Längsrichtung gegenüberliegend angeordneten Verbindungsstellen 23, 24 aus der versetzten Position gemäß Fig. 2 in die ausgerichtete Position gemäß Fig. 1 zurückgedreht werden. In beiden Fällen rotieren also einzelne Umfangssegmente 20 der Gitterstruktur 10 um die Längsachse der Gitterstruktur 10.

Die Expansion der Gitterstruktur 10 erfolgt daher nicht durch eine geradlinig radiale Aufweitung, sondern im Wesentlichen schraubenlinienförmig. Dadurch wird erreicht, dass sich die Gitterstruktur 10 beim Aufweiten innerhalb eines Blutgefäßes 1 schraubenförmig in ein Blutgerinnsel 2 einschneidet, wie in Fig. 5 dargestellt. Die Stege 11, 12, 13, 14 bewegen sich nicht nur in radialer Richtung bezogen auf die Längsachse der Gitterstruktur 10 in das Blutgerinnsel 2 hinein, sondern weisen auch eine Umfangsrichtungskomponente auf, wobei durch die Umfangsbewegung der Stege 11, 12, 13, 14 im Blutgerinnsel 2 ein Hinterschnitt ausgebildet wird, der zu einer verbesserten Anhaftung des Blutgerinnsels 2 an der Gitterstruktur 10 führt. Die Gitterstruktur 10 wird in dem Blutgerinnsel 2 regelrecht verankert, so dass ein ungewolltes Ablösen des Blutgerinnsels 2 von der Gitterstruktur 10 vermieden wird.

### Gitterstruktur

Bei der Gitterstruktur 10 der erfindungsgemäßen medizinischen Vorrichtung bzw. des erfindungsgemäßen Behandlungssystems ist eine kreiszylinderförmige Geometrie der Gitterstruktur 10 vorausgesetzt. Die Umfangssegmente 20 bilden also geschlossene, im Wesentlichen kreiszylinderförmige Ringe. Mehrere Umfangssegmente 20 bilden die Gitterstruktur 10. Durch die kreiszylinderförmige Anordnung der Zellen 15 in den Umfangssegmenten 20 wird verhindert, dass sich die in Umfangsrichtung benachbart angeordneten Verbindungsstellen 21, 22 zueinander versetzen. Vielmehr besteht die geometrische Bedingung, dass die in Umfangsrichtung der Gitterstruktur benachbart angeordneten Verbindungsstellen eines Umfangssegments 20 in einer gemeinsamen Querschnittsebene Q angeordnet sind, die orthogonal auf der Längsachse der Gitterstruktur 10 steht. Tatsächlich erfolgt beim Übergang der Gitterstruktur 10 vom expandierten Zustand in den komprimierten Zustand eine Bewegung der in Längsrichtung benachbart angeordneten Verbindungsstellen 23, 24 entlang des Umfangs der Gitterstruktur 10. Daraus ist erkennbar, dass sich das zweite Verbindungselement 32 beim Übergang vom expandierten Zustand zum komprimierten Zustand der Gitterstruktur 10 stärker verformt als das erste Verbindungselement 31. Da die in Umfangsrichtung benachbart angeordneten Verbindungsstellen 21, 22 auf einer gemeinsamen Querschnittsebene Q angeordnet sind, bewirkt die unterschiedliche Auslenkung bzw. Verformung der Verbindungselemente 31, 32 eine Bewegung der dritten Verbindungsstelle 23 auf dem Umfang der Gitterstruktur 10. Insbesondere rotiert die dritte Verbindungsstelle 23 um einen Rotationsmittelpunkt, der innerhalb der Zelle 15 angeordnet ist. Da die dritte Verbindungsstelle 23 gleichzeitig eine Verbindung zu einem weiteren Umfangssegment 20 der Gitterstruktur 10 bildet, wird das weitere Umfangssegment 20 insgesamt rotiert. Bezogen auf die gesamte Gitterstruktur 10 ergibt sich somit eine Torsion.

Für alle Zustände der Gitterstruktur 10, also für den expandierten Zustand, den komprimierten Zustand und alle möglichen Zwischenzustände, gilt nicht nur, dass die in Umfangsrichtung UR benachbart, angeordneten Verbindungsstellen eine gemeinsame Querschnittsebene Q aufspannen, die zur Längsachse der Gitterstruktur 10 orthogonal ausgerichtet ist, sondern auch, dass der Abstand der in Umfangsrichtung UR benachbart angeordneten Verbindungsstellen innerhalb derselben Querschnittsebene Q gleich ist. Insbesondere entspricht der Abstand zwischen zwei Verbindungsstellen 21, 22, 23, 24, die in Umfangsrichtung UR benachbart angeordnet sind, einem mathematischen Bruchteil des Umfangs der Gitterstruktur 10. Der Abstand zwischen den Verbindungsstellen 21, 22, 23, 24 einer gemeinsamen Querschnittsebene Q ist also einheitlich.

### Nullpunktsgerade in Längsrichtung

Bei den zuvor beschriebenen Ausführungsbeispielen ist vorgesehen, dass in einem expandierten Zustand der Gitterstruktur 10 die in Längsrichtung LR der Gitterstruktur 10 benachbart angeordneten Verbindungsstellen 23, 24 auf einer gemeinsamen Linien angeordnet sind, die sich parallel zur Längsachse der Gitterstruktur 10 erstreckt. Alternativ ist es möglich, dass die in Längsrichtung benachbart angeordneten Verbindungsstellen 23, 24 in einem anderen Zustand der Gitterstruktur auf dieser Nullpunktsgeraden angeordnet sind. Die dritte und vierte Verbindungsstelle 23, 24 können also im expandierten Zustand der Gitterstruktur 10 zueinander versetzt angeordnet bzw. aus der Nullpunktsgeraden ausgelenkt sein. Es ist auch möglich, dass die dritte und vierte Verbindungsstelle 23, 24 sowohl im expandierten Zustand, als auch im komprimierten Zustand aus der Nullpunktsgeraden ausgelenkt sind. Die Anordnung auf der Nullpunktsgeraden kann sich in einem Zwischenzustand der Gitterstruktur 10 einstellen.

Als Nullpunktsgerade wird im Rahmen der Anmeldung die Gerade bezeichnet, die einerseits die in Längsrichtung benachbart angeordneten Verbindungsstellen 23, 24 einer Zelle 15 verbindet und andererseits parallel zur Längsachse der Gitterstruktur 10 bzw. senkrecht zur Querschnittsebene Q ausgerichtet ist. Wenn die in Längsrichtung benachbart angeordneten Verbindungsstellen 23, 24 auf der Nullpunktsgeraden positioniert sind, deckt sich eine Verbindungslinie zwischen den in Längsrichtung benachbart angeordneten Verbindungsstellen 23, 24 mit der Nullpunktsgeraden. Es ist möglich, dass sich die Verbindungslinie zwischen den in Längsrichtung benachbart angeordneten Verbindungsstellen 23, 24 einer Zelle in keinem Zustand der Gitterstruktur 10 mit der Nullpunktsgeraden deckt. Vielmehr kann die Verbindungslinie der in Längsrichtung benachbart angeordneten Verbindungsstellen 23, 24 in jedem Zustand der Gitterstruktur 10 die Nullpunktsgerade unter einem Winkel schneiden.

### Befestigung der Vorrichtung

Die medizinische Vorrichtung nach einem der zuvor genannten Ausführungsbeispiele ist vorzugsweise Teil eines Behandlungssystems, das ferner einen Führungsdraht umfasst, der mit einem proximalen Ende der Vorrichtung, insbesondere der Gitterstruktur 10, verbunden ist. Der Führungsdraht ist längsverschieblich in einem Katheter angeordnet. Mit Hilfe des Führungsdrahts kann die Gitterstruktur 10 bzw. allgemein die medizinische Vorrichtung, im komprimierten Zustand durch den Katheter an einen Behandlungsort geführt werden. Die Bewegung der Gitterstruktur 10 durch den Katheter erfolgt dabei durch Schieben des proximalen Endes des Führungsdrahts in distale Richtung durch den Katheter. Am Behandlungsort wird die Gitterstruktur 10 bzw. allgemein die medizinische Vorrichtung freigesetzt. Das Freisetzen der Gitterstruktur 10 kann auf unterschiedliche Weise erfolgen. Einerseits kann der Führungsdraht weiter in distale Richtung geschoben werden, wobei der Katheter ortsfest gehalten wird. Alternativ kann der Führungsdraht ortsfest gehalten werden und der Katheter in proximale Richtung zurückgezogen werden, um die Gitterstruktur 10 aus dem Katheter zu entlassen. In beiden Fällen erfolgt die Entlassung der Gitterstruktur 10 durch eine translatorische Relativbewegung zwischen dem Führungsdraht und dem Katheter.

Durch Wegfall des äußeren Zwanges, den der Katheter auf die Gitterstruktur ausübt und der diese im komprimierten Zustand hält, expandiert die Gitterstruktur 10. Dazu weist die Gitterstruktur 10 vorzugsweise ein selbstexpandierendes Material auf. Derartige Materialien sind beispielsweise Formgedächtnislegierungen, insbesondere Nickel-Titan-Legierungen.

Aufgrund der zuvor beschriebenen Konstruktion und Eigenschaften der Gitterstruktur 10 erfolgt allein durch die radiale Expansion der Gitterstruktur 10 eine Torsion. Die Drehung der Gitterstruktur 10 wird also durch die translatorische Relativbewegung zwischen der Gitterstruktur 10 bzw. den damit verbundenen Führungsdraht und dem Katheter bewirkt. Die translatorische Relativbewegung zwischen Führungsdraht und Katheter ist vergleichsweise einfach steuerbar im Gegensatz zu einer manuellen Drehung, die am proximalen Ende des Führungsdrahts durchgeführt wird. Bei der manuellen Drehung des Führungsdrahts erfolgt zunächst eine Torsion des Führungsdrahts, so dass die eigentliche Verdrehung der distal angeordneten medizinischen Vorrichtung bzw. Gitterstruktur 10 verzögert erfolgt. Dies ist bei einer translatorischen Bewegung nicht gegeben, so dass die Handhabbarkeit des erfindungsgemäßen Behandlungssystems verbessert ist.

### Bezugszeichenliste

- 1.: Blutgefäß
- 2: Blutgeririnsel
- 10: Gitterstruktur
- 11: erster Steg
- 12: zweiter Steg
- 13: dritter Steg
- 14: vierter Steg
- 15: Zelle
- 16: Unterzelle
- 20: Umfangssegment
- 21: erste Verbindungsstelle
- 22: zweite Verbindungsstelle
- 23: dritte Verbindungsstelle
- 24: vierte Verbindungsstelle
- 30: Zwischensteg
- 31: erstes Verbindungselement
- 32: zweites Verbindungselement
- 33: Spitze
- 41: erste Kopplungsstelle
- 42, 43: zweite Kopplungsstellen

## Patentansprüche

1. Medizinische Vorrichtung mit einer komprimierbaren und expandierbaren, kreiszylinderförmigen Gitterstruktur (10), die geschlossene Zellen (15) umfasst, wobei die Zellen (15) durch jeweils wenigstens vier Stege (11, 12, 13, 14) begrenzt sind, die an Verbindungsstellen (21, 22, 23, 24) miteinander gekoppelt sind, wobei die Verbindungsstellen (21, 22, 23, 24) im expandierten Zustand der Gitterstruktur (10) eine rautenförmige Grundform der Zelle (15) bilden und
wenigstens eine Zelle (15) einen Zwischensteg (30) aufweist **dadurch gekennzeichnet, dass** der Zwischensteg (30) sich zwischen zwei in Längsrichtung der Gitterstruktur (10) direkt benachbarten und miteinander verbundenen Stege (12, 13) der Zelle (15) durch die Zelle (15) erstreckt und eine Spitze (33) bildet, die im Wesentlichen in Umfangsrichtung der Gitterstruktur (10) zeigt.

2. Vorrichtung nach Anspruch 1,
**dadurch gekennzeichnet, dass**
der Zwischensteg (30) mit den beiden direkt benachbarten und miteinander verbundenen Stege (11, 12, 13, 14) eine Unterzelle (16) bildet, die im Wesentlichen dieselbe Form wie die übergeordnete Zelle (15) aufweist.

3. Vorrichtung nach Anspruch 1 oder 2,
**dadurch gekennzeichnet, dass**
der Zwischensteg (30) durch zwei Verbindungselemente (31, 32) gebildet ist, die im Bereich der Spitze (33) miteinander verbunden sind, wobei die Verbindungselemente (31, 32) unterschiedlich flexibel sind derart, dass der Zwischensteg (30) beim Übergang der Gitterstruktur (10) vom radial komprimierten Zustand in den radial expandierten Zustand zumindest abschnittsweise aus der Umfangsebene der Gitterstruktur (10) ausgelenkt wird.

4. Vorrichtung, nach Anspruch 3,
**dadurch gekennzeichnet, dass**
die Verbindungselemente (31, 32) jeweils unterschiedliche Länge aufweisen.

5. Vorrichtung nach einem der Ansprüche 3 oder 4,
**dadurch gekennzeichne**t, dass
das Verbindungselement (32) mit höherer Flexibilität S-förmige oder mäanderförmige Teile aufweist, wobei das Verbindungselement (31) mit niedrigerer Flexibilität geradlinig ausgebildet ist.

6. Vorrichtung nach einem der Ansprüche 3 bis 5,
**dadurch gekennzeichnet, dass**
das Verbindungselement (32) mit höherer Flexibilität eine Breite aufweist, die kleiner als die Breite des Verbindungselements (31) mit niedrigerer Flexibilität ist.

7. Vorrichtung nach einem der Ansprüche 3 bis 6,
**dadurch gekennzeichnet, dass**
das Verbindungselement (32) mit höherer Flexibilität Biegestellen, insbesondere Verjüngungen oder Durchbrüche, aufweist.

8. Vorrichtung nach einem der Ansprüche 3 bis 7,
**dadurch gekennzeichnet, dass**
ein oder beide Verbindungselemente (31, 32) einen Schneidebereich aufweisen, der sich bei einer Rotation in Umfangsrichtung UR bewegt.

9. Vorrichtung nach einem der Ansprüche 3 bis 8,
**dadurch gekennreichnet,** dass
die Verbindungselemente (31, 32) an einer ersten Kopplungsstelle (41) miteinander verbunden, insbesondere stoffschlüssig verbunden sind.

10. Vorrichtung nach Anspruch 9,
**dadurch gekennzeichnet, dass**
die Verbindungselemente (31, 32) an zweiten Kopplungsstellen (42, 43) mit den benachbarten Stege verbunden sind, wobei im expandierten Zustand eine viereckige Grundform, insbesondere eine konvexe viereckige Grundform, durch die zweiten Kopplungsstellen (42, 43), die erste Kopplunglungsstelle (41) zwischen den Verbindungselementen (31, 32), und die Verbindungsstelle (21) zwischen den benachbarten Stegen gebildet wird.

11. Vorrichtung nach einem der vorhergehenden Ansprüche,
**dadürch gekennzeichnet,** dass
die in Längsrichtung LR der Gitterstruktur (10) gegenüberliegend angeordneten Verbindungsstellen (23, 24) einen unterschiedlichen Vernetzungsgrad (α, β) beim Übergang der Gitterstruktur (10) vom expandierten in den komprimierten Zustand aufweisen.

12. Vorrichtung nach einem der vorhergehenden Ansprüche,
**dadurch gekennzeichnet, dass**
in Umfangsrichtung UR benachbart angeordnete Verbindungsstellen (23, 24) eines Umfangssegments (20) eine gemeinsame Querschnittsebene Q der Gitterstruktur (10) aufspannen, die orthogonal zur Längsachse der Gitterstruktur (10) angeordnet ist, wobei sich die in Umfangsrichtung UR benachbart angeordneten Verbindungsstellen (23, 24) beim Übergang der Gitterstruktur (10) vom expandierten Zustand in den komprimierten Zustand in der Querschnittsebene Q bewegen.

13. Behandlungssystem mit einer medizinischen Vorrichtung nach einem der vorhergehenden Ansprüche und mit einem Katheter, in dem ein Führungselement längsverschieblich angeordnet ist, wobei das Führungselement fest, insbesondere drehfest, mit einem axialen Ende der Gitterstruktur (10) der medizinischen Vorrichtung verbunden ist.

## Claims

1. A medical device with a compressible and expandable circular cylindrical lattice structure (10) comprising closed cells (15), wherein the cells (15) are each delimited by at least four webs (11, 12, 13, 14) that are connected to one another at connection points (21, 22, 23, 24), wherein the connection points (21, 22, 23, 24) form a diamond-shaped basic shape of the cell (15) in the expanded state of the lattice structure (10) and at least one cell (15) has an intermediate web (30), **characterized in that** the intermediate web (30) extends through the cell (15) between two webs (12, 13) of the cell (15) which are directly adjacent in the longitudinal direction of the lattice structure (10) and are connected to one another, and forms a tip (33) which substantially points in the circumferential direction of the lattice structure (10).

2. The device according to claim 1, **characterized in that** the intermediate web (30), together with the two webs (11, 12, 13, 14) that are directly adjacent and connected to one another, forms a sub-cell (16) that has substantially the same shape as the superordinated cell (15).

3. The device according to claim 1 or 2, **characterized in that** the intermediate web (30) is formed by two connection elements (31, 32) that are connected to one another in the region of the tip (33), wherein the connection elements (32, 32) are differently flexible in such a manner that the intermediate web (30), during the transition of the lattice structure (10) from the radially compressed state into the radially expanded state, is deflected out of the circumferential plane of the lattice structure (10), at least in sections.

4. The device according to claim 3, **characterized in that** the connection elements (31, 32) each have different lengths.

5. The device according to any one of claims 3 or 4, **characterized in that** the connection element (32) having higher flexibility has S-shaped or meander-shaped parts, wherein the connection element (31) having lower flexibility is formed straight.

6. The device according to any one of claims 3 to 5, **characterized in that** the connection element (32) having higher flexibility has a width that is smaller than the width of the connection element (31) having lower flexibility.

7. The device according to any one of claims 3 to 6, **characterized in that** the connection element (32) having higher flexibility has bending points, in particular taperings or openings.

8. The device according to any one of claims 3 to 7, **characterized in that** one or both connection elements (31, 32) have a cutting region which moves in the circumferential direction UR when rotating.

9. The device according to any one of claims 3 to 8, **characterized in that** the connection elements (31, 32) are connected to one another, in particular firmly bonded, at a first coupling point (41).

10. The device according to claim 9, **characterized in that** the connection elements (31, 32) are connected to the adjacent webs at second coupling points (42, 43), wherein in the expanded state, a rectangular basic shape, in particular a convex rectangular basic shape is formed by the second coupling points (42, 43), the first coupling point (41) between the connection elements (31, 32), and the connection point (21) between the adjacent webs.

11. The device according to any one of the preceding claims, **characterized in that** the connection points (23, 24) arranged opposite to one another in the longitudinal direction LR of the lattice structure (10) have a different degree of displacement (α, β) during the transition of the lattice structure (10) from the expanded into the compressed state.

12. The device according to any one of the preceding claims, **characterized in that** connection points (23, 24) of a circumferential segment (20), which connection points are arranged adjacent in the circumferential direction UR, span a common cross-sectional plane Q of the lattice structure (10) which is arranged orthogonal to the longitudinal axis of the lattice structure (10), wherein the connection points (23, 24) arranged adjacent in the circumferential direction UR move in the cross-sectional plane Q during the transition of the lattice structure (10) from the expanded state into the compressed state.

13. A treatment system with a medical device according to any one of the preceding claims, and with a catheter, in which a guide element is arranged in a longitudinally displaceable manner, wherein the guide element is fixedly connected, in particular rotationally fixedly connected, to an axial end of the lattice structure (10) of the medical device.

## Revendications

1. Dispositif médical doté d'une structure de grille de forme cylindrique compressible et expansible (10), qui comprend des cellules fermées (15), les cellules (15) étant limitées respectivement par au moins quatre traverses (11, 12, 13, 14) qui sont couplées entre elles par des points de connexion (21, 22, 23, 24), les points de connexion (21, 22, 23, 24) formant, à l'état expansé de la structure de grille (10), une forme de base en forme de losange de la cellule (15) et au moins une cellule (15) présentant une traverse intermédiaire (30), **caractérisé en ce que** la traverse intermédiaire (30) s'étend entre deux traverses directement voisines dans le sens longitudinal de la structure de grille (10) et connectées entre elles (12, 13) de la cellule (15) à travers la cellule (15) et forme une pointe (33) qui est orientée sensiblement dans le sens circonférentiel de la structure de grille (10).

2. Dispositif selon la revendication 1, **caractérisé en ce que** la traverse intermédiaire (30) constitue avec les deux traverses directement voisines et reliées entre elles (11, 12, 13, 14) une sous-cellule (16) qui présente sensiblement la même forme que la cellule d'ordre supérieur (15).

3. Dispositif selon la revendication 1 ou 2, **caractérisé en ce que** la traverse intermédiaire (30) est constituée par deux éléments de connexion (31, 32) qui sont reliés entre eux au niveau de la pointe (33), les éléments de connexion (31, 32) étant diversement souples de manière à ce que la traverse intermédiaire (30), à la transition de la structure de grille (10) de l'état radialement comprimé à l'état radialement expansé, soit déviée du moins par endroits du plan circonférentiel de la structure de grille (10).

4. Dispositif selon la revendication 3, **caractérisé en ce que** les éléments de connexion (31, 32) présentent respectivement des longueurs différentes.

5. Dispositif selon une des revendications 3 ou 4, **caractérisé en ce que** l'élément de connexion (32) à souplesse supérieure présente des parties en forme de S ou en forme de méandres, l'élément de connexion (31) à souplesse inférieure ayant une conformation rectiligne.

6. Dispositif selon une des revendications 3 à 5, **caractérisé en ce que** l'élément de connexion (32) à souplesse supérieure présente une largeur qui est inférieure à la largeur de l'élément de connexion (31) à souplesse inférieure.

7. Dispositif selon une des revendications 3 à 6, **caractérisé en ce que** l'élément de connexion (32) à souplesse supérieure présente des points de flexion, en particulier des rétrécissements ou des percées.

8. Dispositif selon une des revendications 3 à 7, **caractérisé en ce qu'**un ou deux éléments de connexion (31, 32) présente une zone de coupe qui se déplace en cas de rotation dans le sens circonférentiel UR.

9. Dispositif selon une des revendications 3 à 8, **caractérisé en ce que** les éléments de connexion (31, 32) sont reliés entre eux à un point de couplage (41), en particulier par correspondance de matière.

10. Dispositif selon la revendication 9, **caractérisé en ce que** les éléments de connexion (31, 32) sont reliés à deux seconds point de couplage (42, 43) aux traverses voisines, sachant que, à l'état expansé, une forme de base quadrangulaire, en particulier une forme de base quadrangulaire convexe, est formée par les seconds points de couplage (42, 43), le premier point de couplage (41) entre les éléments de connexion (31, 32) et le point de liaison (21) entre les traverses voisines.

11. Dispositif selon une des revendications précédentes, **caractérisé en ce que**, les points de liaison (23, 24) disposés opposés dans le sens longitudinal LR de la structure de grille (10) présentent un niveau de transfert différent (α, β) à la transition de la structure de grille (10) de l'état expansé à l'état comprimé.

12. Dispositif selon une des revendications précédentes, **caractérisé en ce que** les points de liaison (23, 24) disposés adjacents dans le sens circonférentiel UR d'un segment circonférentiel (20) sous-tendent un plan de section transversale Q commun (10) qui est disposé orthogonalement à l'axe longitudinal de la structure de grille (10), les points de liaison (23, 24) disposés adjacents dans le sens circonférentiel UR se déplaçant, à la transition de la structure de grille (10) de l'état expansé à l'état comprimé, dans le plan de section transversale Q.

13. Système de traitement doté d'un dispositif médical selon une des revendications précédentes et d'un cathéter dans lequel un élément de guidage est disposé de manière déplaçable en longueur, l'élément de guidage étant relié fixement, en particulier fixement en rotation, à une extrémité axiale de la structure de grille (10) du dispositif médical.
